(19) Europäisches Patentamt
European Patent Office
Office européen des brevets

(11) EP 4 613 256 A1

(12) EUROPEAN PATENT APPLICATION
published in accordance with Art. 153(4) EPC

(43) Date of publication:
**10.09.2025 Bulletin 2025/37**

(21) Application number: **23885773.4**

(22) Date of filing: **31.10.2023**

(51) International Patent Classification (IPC):
***A61K 6/831*** *(2020.01)* ***A61C 13/00*** *(2006.01)*
***A61C 13/23*** *(2006.01)* ***A61K 6/887*** *(2020.01)*

(52) Cooperative Patent Classification (CPC):
**A61C 13/00; A61C 13/0025; A61K 6/831; A61K 6/887**

(86) International application number:
**PCT/JP2023/039313**

(87) International publication number:
**WO 2024/096019 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.10.2022 JP 2022175144**

(71) Applicant: **Kuraray Noritake Dental Inc.**
**Kurashiki-shi, Okayama 710-0801 (JP)**

(72) Inventors:
- **KAWANA, Mariko**
  **Tainai-shi, Niigata 959-2653 (JP)**
- **OKADA, Keishu**
  **Tokyo 100-0004 (JP)**
- **NOJIRI, Yamato**
  **Tokyo 100-0004 (JP)**
- **Takei, Mitsuru**
  **Tainai-shi, Niigata 959-2653 (JP)**

(74) Representative: **D Young & Co LLP**
**3 Noble Street**
**London EC2V 7BQ (GB)**

# (54) DENTAL ADHESIVE COMPOSITION

(57) A dental adhesive composition containing a polymerizable monomer (A) having an acidic group and a photoredox catalyst (B) having a singlet excited state reduction potential $E^{S1}_{red}$ of 1.5 V vs SCE or more.

EP 4 613 256 A1

## Description

Technical Field

The present invention relates to a dental adhesive composition.

Background Art

For restorative remedy of missing parts of the tooth substance damaged by caries, fractures, and the like, filling restoration materials, such as filling composite resins, and dental crown restoration materials, such as metal alloys, ceramics, resin materials, have been widely used. Dental bonding materials have been mainly used for adhering the filling restoration material and the tooth substance, and dental cement has been mainly used for adhering the dental crown restoration material and the tooth substance. Dental self-adhesive composite resins, which have both the functions of the filling restoration material and the dental bonding material, are being used in recent years. As these dental adhesive compositions having adhesion to the tooth substance, such as dental bonding material, dental cement, and dental self-adhesive composite resin, resin based compositions containing polymerizable monomers, polymerization initiators, fillers, and other components have been generally used.

Among the dental adhesive compositions, the dental bonding materials are generally liquid polymerizable monomer-containing compositions containing polymerizable monomers, polymerization initiators, stabilizers, and the like dissolved therein. On the other hand, the dental self-adhesive resin cement and the dental self-adhesive composite resin are both paste-like compositions, each of which are generally produced by mixing a liquid polymerizable monomer-containing composition containing polymerizable monomers, polymerization initiators, stabilizers, and the like dissolved therein, with powder fillers and the like. Dental adhesive compositions are filled in containers and delivered to dentists as users, and are required to maintain the prescribed performances within their expiration date.

(Meth)acrylates have been generally used as polymerizable monomers contained in dental bonding materials, dental self-adhesive resin cement, and dental self-adhesive composite resin.

As described below, dental bonding materials, dental self-adhesive resin cement, and dental self-adhesive composite resin contain polymerizable monomers having one or more acidic groups, such as phosphoric acid group or carboxy group, to impart adhesion to tooth substance or prosthesises.

As adhesion system using dental bonding materials, so-called acid etching type (total etching type) adhesion system has been generally used, in which the surface of the tooth substance is subjected to an etching treatment by coating an acid etching material, such as a phosphoric acid aqueous solution, thereon, and then coated with the bonding material as an adhesive, and then the dental restorative material is coated thereon, so as to adhere the tooth substance and the dental restorative material. On the other hand, adhesion systems that do not use the acid etching material include so-called self-etching type adhesion system. The mainstream of this adhesion system has been the two-step adhesion system, in which a self-etching primer containing acidic monomers, hydrophilic monomers, and water is coated on the surface of tooth substance, and then without water rinsing, the bonding material containing crosslinking monomers and polymerization initiators is coated thereon, but in recent years, one-step adhesion system using one-component type dental bonding materials having both the functions of the self-etching primer and the bonding material (i.e., a one-component type dental bonding material) are being generally used. One-component type dental bonding materials generally contain acidic monomers, hydrophilic monomers, crosslinking monomers, and the like as monomer components.

In the restoration methods described above, while two materials, i.e., dental bonding materials and dental filling restoration materials (dental filling composite resin), are used, dental self-adhesive composite resin, which are dental filling composite resin having adhesion imparted thereto, are developed in recent years, and are being subjected to practical use as materials that reduce the operation steps of the restorative remedy by omitting the use of dental bonding materials. The dental self-adhesive composite resin contain the polymerizable monomers having one or more acidic groups, which have been conventionally used in the dental bonding materials, to impart the adhesion to tooth substance, in addition to the components of the ordinary dental filling composite resin, e.g., the polyfunctional polymerizable monomer and fillers for imparting the mechanical strength, and polymerization initiators for enhancing the curability.

Dental resin cement has generally used a system using one or more pretreatment materials (i.e., primers or bonding materials) and a two-paste type dental resin cement. This system is referred to as a pretreatment material combination type dental resin cement. In the pretreatment material combination type dental resin cement, it is necessary that before adhering a dental crown material to the tooth substance, pretreatment materials containing one or more adhesive monomers are used for performing simultaneously the removal of the smear layer on the surface of the tooth and the permeation of the monomer to the collagen layer, and then the tooth substance having the monomer permeated thereto and the prosthesis are adhered through curing of the dental resin cement. In recent years, for reducing the burden on the patients by speeding up the dental treatment, there is a demand of simplifying the dental treatment operation, and cement

capable of adhering the tooth substance and a prosthesis without use of pretreatment materials, i.e., dental self-adhesive resin cement capable of performing all demineralization, permeation, and curing at one time, is being used.

The dental adhesive compositions described above are demanded to have enhanced adhesion to tooth substance, particularly to dentine. The necessary functions for providing adhesion strength to the dentine with a dental adhesive composition include the "demineralization function" of dissolving the dentine surface with an acidic component, the "permeation function" of permeating the monomer component to the collagen layer of the dentine, and the "curing function" of forming a hybrid layer of the permeated and cured monomer component and the collagen (which may be hereinafter referred to as a resin permeation layer). In these elemental functions, the enhancement of the curability of the resin permeation layer is particularly important for enhancing the adhesion.

Polymerization initiators used for curing these dental adhesive compositions may include photopolymerization initiator systems capable of performing polymerization curing with light, such as visible light, and redox type chemical polymerization initiator systems containing oxidizing agents, reducing agents, and the like. The photopolymerization initiators can be used in the one-component or one-material type composition, and can perform adhesion of the composition through polymerization and curing by irradiating light, such as blue light, with a dental irradiator after applying the composition. In the case where the dental filling restoration material is adhered with the dental bonding material, the adhesion to the tooth substance can be performed through light irradiation to the dental bonding material and the dental filling restoration material. On the other hand, the chemical polymerization initiators, i.e., the combination of oxidizing agents and reducing agents, are necessarily packaged separately, for example, into the first part containing oxidizing agents and the second part containing reducing agents, and delivered to dentists as users in the form of a divided dental adhesive compositions. Dentists necessarily mix the first part and the second part immediately before use, through which radicals are generated through the redox reaction to perform the polymerization curing of the dental adhesive composition even in the region where no light reaches, and thereby a crown restoration materials and the like can be adhered to tooth substance.

PTL 1 describes a dental bonding material. PTL 2 describes dental self-adhesive resin cement. PTL 3 describes a polymerization initiator technique in a dental self-adhesive composite resin.

PTL 1 relates to the invention, in which the polymerization curability of the resin permeation layer can be enhanced by applying a bisacylphosphine oxide based polymerization initiator to the dental bonding material, and thereby a high adhesion strength can be obtained. PTL 2 describes a chemical polymerization initiator system that is effective for enhancing the adhesion strength of the dental self-adhesive resin cement to tooth substance. PTL 3 describes a dental self-adhesive composite resin having an initiator system including a persulfate salt, a transition metal, and a photoinitiator.

Citation List

Patent Literatures

PTL 1: WO 2008/087981
PTL 2: WO 2010/106903
PTL 3: WO 2013/082337

Summary of Invention

Technical Problem

As a result of the investigation by the present inventors, the use of the polymerization initiator technique described in PTL 1 can indeed enhance the polymerization curability to the dental bonding material, but has room for improvement in the adhesion strength to the tooth substance in the one-component type dental bonding material. The chemical polymerization initiator technique in the dental adhesive composition described in PTL 2 is effective for enhancing the adhesion strength to the tooth substance without light irradiation, but there is no investigation on the enhancement of the adhesion strength with light irradiation. In recent years, however, light-transmitting prostheses are being increased due to the demand of enhanced esthetics of the dental crown materials. This market environmental change of the dental crown materials increases the demand of the photopolymerization methods for the curing method of the dental resin cement in recent years, in addition to the chemical polymerization methods as the ordinary mainstream, and therefore it is necessary to investigate the enhancement of the adhesion strength of the dental resin cement through photopolymerization. The technique described in PTL 3 exhibits adhesion strength to the tooth substance in the dental self-adhesive composite resin, but is inferior in adhesion strength to the adhesion strength in the case using the bonding material in combination, and there is room of improvement.

The dental adhesive compositions are also demanded to maintain the high adhesion to the tooth substance even after storing, in addition to the adhesion strength.

An objective of the present invention is to provide a dental adhesive composition that is excellent in adhesion to tooth substance in photocuring, and also can maintain the high adhesion to tooth substance even after storing.

Solution to Problem

As a result of the earnest investigations by the present inventors on the dental adhesive composition that is excellent in adhesion to tooth substance in photocuring, and also is less decreased in the performances during long-term storage, it has been found that a dental adhesive composition containing a photoredox catalyst that satisfies a singlet excited state reduction potential $E^{S1}{}_{red}$ of a certain value or more shows a significantly high adhesion strength to tooth substance, and maintains the high adhesion to the tooth substance even after storing, and after further accumulating investigations, the present invention has been completed.

Specifically, the present invention encompasses the following inventions.

[1] A dental adhesive composition containing a polymerizable monomer (A) having an acidic group and a photoredox catalyst (B) having a singlet excited state reduction potential $E^{S1}{}_{red}$ of 1.5 V vs SCE or more.

[2] The dental adhesive composition according to the item [1], in which the photoredox catalyst (B) has a negative singlet excited state oxidation potential $E^{S1}{}_{ox}$.

[3] The dental adhesive composition according to the item [1] or [2], in which the photoredox catalyst (B) is at least one kind selected from the group consisting of compounds represented by the following formulae (1), (2), and (3):

[Chem. 1]

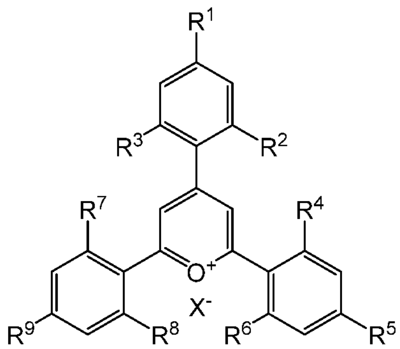

(1)

in which in the formula (1), $R^1$, $R^2$, $R^3$, $R^4$, $R^5$, $R^6$, $R^7$, $R^8$, and $R^9$ each independently represent a hydrogen atom, an alkyl group having 1 to 10 carbon atoms, an alkoxy group having 1 to 10 carbon atoms, or a halogen atom, and $X^-$ represents $BF_4^-$, $PF_6^-$, $ClO_4^-$, or $HSO_4^-$,

[Chem. 2]

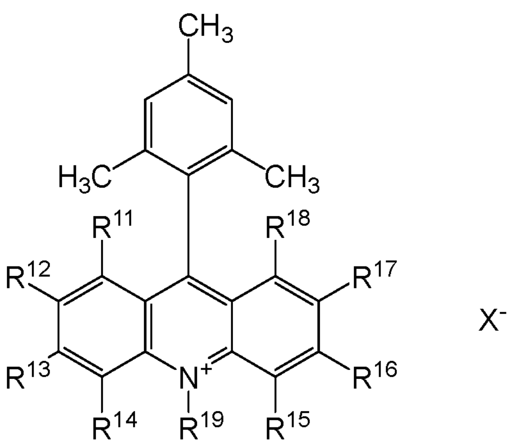

(2)

in the formula (2), $R^{11}$, $R^{13}$, $R^{14}$, $R^{15}$, $R^{16}$, and $R^{18}$ each independently represent a hydrogen atom or an alkyl group

having 1 to 10 carbon atoms, $R^{12}$, $R^{17}$, and $R^{19}$ each independently represent a hydrogen atom, an alkyl group having 1 to 10 carbon atoms, or an aryl group having 6 to 30 carbon atoms, and $X^-$ represents $BF_4^-$, $PF_6^-$, $ClO_4^-$, or $HSO_4^-$,

[Chem. 3]

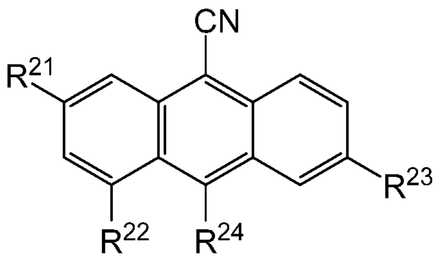

(3)

in the formula (3), $R^{21}$, $R^{22}$, $R^{23}$, and $R^{24}$ each independently represent a hydrogen atom, an alkyl group having 1 to 10 carbon atoms, an alkoxy group having 1 to 10 carbon atoms, a carboxylic acid group having 1 to 10 carbon atoms or an ester thereof, a hydroxy group, a cyano group, or a halogen atom.

[4] The dental adhesive composition according to the item [3], in which in the formula (1), $R^1$, $R^2$, and $R^3$ each independently represent an alkyl group having 1 to 4 carbon atoms.

[5] The dental adhesive composition according to the item [3] or [4], in which in the formula (1), $R^1$, $R^2$, and $R^3$ each independently represent a methyl group, an isopropyl group, or a tert-butyl group.

[6] The dental adhesive composition according to any one of the items [1] to [5], in which the photoredox catalyst (B) has an absorption spectrum including a range of 400 nm or more and 500 nm or less.

[7] The dental adhesive composition according to any one of the items [1] to [6], in which the dental adhesive composition further contains a polymerizable monomer (C) having no acidic group.

[8] The dental adhesive composition according to any one of the items [1] to [7], in which the dental adhesive composition further contains an organic peroxide (D).

[9] The dental adhesive composition according to any one of the items [1] to [8], in which the dental adhesive composition further contains a reducing agent (E).

[10] The dental adhesive composition according to any one of the items [1] to [9], in which the dental adhesive composition further contains a phosphine compound (F) having an electron withdrawing group.

[11] The dental adhesive composition according to any one of the items [1] to [10], in which the dental adhesive composition further contains a photopolymerization initiator (G).

[12] The dental adhesive composition according to any one of the items [1] to [11], in which the dental adhesive composition is a one-component type dental bonding material.

[13] The dental adhesive composition according to any one of the items [1] to [11], in which the dental adhesive composition is a dental self-adhesive composite resin.

[14] The dental adhesive composition according to any one of the items [1] to [11], in which the dental adhesive composition is dental self-adhesive resin cement.

[15] The dental adhesive composition according to any one of the items [1] to [11], in which the dental adhesive composition is a kit including a one-component type dental bonding material containing the photoredox catalyst (B), and a dental two-paste type composition.

[16] The dental adhesive composition according to any one of the items [1] to [11], in which the dental adhesive composition is a kit including a one-component type dental bonding material containing the photoredox catalyst (B), and a dental one-paste type composition.

## Advantageous Effects of Invention

The present invention can provide a dental adhesive composition that is excellent in adhesion to tooth substance in photocuring, and also can maintain the high adhesion to the tooth substance even after storing.

## Description of Embodiments

The present invention will be described in detail with reference to embodiments below.

In the description herein, the upper limit values and the lower limit values of the numerical ranges (e.g., the contents of the components, and the values and the properties calculated from the components) can be optionally combined.

Specifically, in the description herein, the upper limit values and the lower limit values described in a stepwise

manner for the numerical ranges can each be independently combined. For example, with reference to the description "preferably 10 to 90, and more preferably 30 to 60" for the same item, a range of "10 to 60" can be derived from the "preferred lower limit value (10)" and the "more preferred upper limit value (60)".

For the numerical ranges, for example, based on the description "preferably 10 to 90, and more preferably 30 to 60", only the lower limit value can be defined as "10 or more" or "30 or more" with no particular definition of the upper limit value. Similarly, only the upper limit value can be defined as "90 or less" or "60 or less" with no particular definition of the lower limit value.

A numerical range simply defined as "10 to 90" means a range of 10 or more and 90 or less unless otherwise indicated.

As similar to as described above, with reference to the description "preferably 10 or more, and more preferably 30 or more" and the description "preferably 90 or less, and more preferably 60 or less", a range of "10 or more and 60 or less" can be derived from the "preferred lower limit value (10)" and the "more preferred upper limit value (60)". As similar to as described above, only the lower limit value can be defined as "10 or more" or "30 or more", and similarly, only the upper limit value can be defined as "90 or less" or "60 or less".

The (meth)acryloyl group means an acryloyl group or a methacryloyl group, which is similarly applied to the analogous terms.

In the description herein, the "storage stability" means that the high adhesion to tooth substance is maintained even after storing. Therefore, in the case where the high adhesion is still achieved even through the adhesion is decreased due to long-term storage, it can be said that the "storage stability" is high.

The dental adhesive composition of the present invention contains a polymerizable monomer (A) having an acidic group and a photoredox catalyst (B) having a singlet excited state reduction potential $E^{S1}_{red}$ of 1.5 V vs SCE or more.

The mechanism of exerting the effects of the present invention by the dental adhesive composition of the present invention is not clear, but can be estimated as follows.

The compound having a singlet excited state reduction potential $E^{S1}_{red}$ of a certain value or more is excited through light irradiation, and functions as a strong oxidizing agent. In the case where the dental adhesive composition contains the compound, redox reaction occurs at the interface between tooth substance and the dental adhesive composition in curing the dental adhesive composition through irradiation of light, such as visible light, so as to generate a large amount of radicals. It is estimated that the large amount of radicals largely accelerate the polymerization curing reaction at the adhesion interface, and thereby the adhesion to tooth substance is enhanced.

Among the photoredox catalysts, many of cationic substances are decomposed by receiving nucleophilic attack through coexistence with a basic substance, and thereby the storage stability may be decreased in some cases. In the present invention, the photoredox catalyst coexists with the acidic group-containing polymerizable monomer, and thereby the high storage stability can be obtained even with the cationic photoredox catalyst. Furthermore, it has been known that an ordinary oxidizing agent tends to decompose under an acidic condition. However, the "photoredox catalyst" having a singlet excited state reduction potential $E^{S1}_{red}$ of a certain value or more remains stable until exposed to light irradiation. Upon excitation, it functions as an oxidizing agent, enabling the incorporation of an otherwise unstable oxidizing agent into the dental adhesive composition in a stabilized form. In addition to the above, an oxidizing agent and a reducing agent, which normally causes redox reaction by mixing, can be allowed to coexist in a stable form in the same material. The high storage stability can be achieved even in the case where, for example, the photoredox catalyst and a reducing agent are blended in the same material.

It can be estimated that the effects of the present invention are achieved through the estimated mechanism described above.

The components contained in the dental adhesive composition of the present invention each will be described below.

[Polymerizable Monomer (A) having Acidic Group]

The dental adhesive composition of the present invention contains the polymerizable monomer (A) having an acidic group. The polymerizable monomer (A) having an acidic group is an essential component for allowing the dental adhesive composition of the present invention to exhibit adhesion, and has a function of demineralizing the tooth substance.

The polymerizable monomer (A) having an acidic group is a polymerizable monomer that has at least one acidic group, such as a phosphoric acid group, a phosphonic acid group, a pyrophosphoric acid group, a thiophosphoric acid group, a carboxylic acid group, and a sulfonic acid group, and has at least one polymerizable group, such as an acryloyl group, a methacryloyl group, an acrylamide group, and a methacrylamide group. The polymerizable monomer (A) having an acidic group is preferably monofunctional with one of an acryloyl group, a methacryloyl group, an acrylamide group, and a methacrylamide group as the polymerizable group from the standpoint of the adhesion to the tooth substance. Specific examples thereof include the following.

Examples of the polymerizable monomer having a phosphoric acid group include a monofunctional (meth) acrylate compound having a phosphoric acid group, such as 2-(meth)acryloyloxyethyl dihydrogen phosphate, 3-(meth) acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth) acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyeicosyl dihydrogen phosphate, 2-(meth)acryloyloxyethylphenyl hydrogen phosphate, 2-(meth)acryloyloxyethyl-2-bromoethyl hydrogen phosphate, 2-(meth)acryloyloxyethyl-(4-methoxyphenyl) hydrogen phosphate, and 2-(meth)acryloyloxypropyl-(4-methoxyphenyl) hydrogen phosphate, and hydrochlorides, alkali metal salts, ammonium salts, and amine salts thereof; and a bifunctional (meth)acrylate compound having a phosphoric acid group, such as bis[2-(meth)acryloyloxyethyl] hydrogen phosphate, bis[4-(meth)acryloyloxybutyl] hydrogen phosphate, bis[6-(meth)acryloyloxyhexyl] hydrogen phosphate, bis[8-(meth)acryloyloxyoctyl] hydrogen phosphate, bis[9-(meth)acryloyloxynonyl] hydrogen phosphate, bis [10-(meth)acryloyloxydecyl] hydrogen phosphate, and 1,3-di(meth)acryloyloxypropyl dihydrogen phosphate, and hydrochlorides, alkali metal salts, ammonium salts, and amine salts thereof.

Among these, 10-(meth)acryloyloxydecyl dihydrogen phosphate and 1,3-di(meth)acryloyloxypropyl dihydrogen phosphate are preferably used.

Examples of the polymerizable monomer having a phosphonic acid group include 2-(meth)acryloyloxyethyl phenylphosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexyl phosphonoacetate, and 10-(meth)acryloyloxydecyl phosphonoacetate, and hydrochlorides, alkali metal salts, ammonium salts, and amine salts thereof.

Examples of the polymerizable monomer having a pyrophosphoric acid group include bis[2-(meth)acryloyloxyethyl] pyrophosphate, bis[4-(meth)acryloyloxybutyl] pyrophosphate, bis[6-(meth)acryloyloxyhexyl] pyrophosphate, bis [8-(meth)acryloyloxyoctyl] pyrophosphate, and bis[10-(meth)acryloyloxydecyl] pyrophosphate, and hydrochlorides, alkali metal salts, ammonium salts, and amine salts thereof.

Examples of the polymerizable monomer having a thiophosphoric acid group include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth) acryloyloxyundecyl dihydrogen thiophosphate, 12-(meth)acryloyloxydodecyl dihydrogen thiophosphate, 16-(meth)acryloyloxyhexadecyl dihydrogen thiophosphate, and 20-(meth)acryloyloxyeicosyl dihydrogen thiophosphate, and hydrochlorides, alkali metal salts, and ammonium salts thereof.

Examples of the polymerizable monomer having a carboxylic acid group include (meth)acrylic acid, 4-[2-[(meth) acryloyloxy]ethoxycarbonyl]phthalic acid, 4-(meth)acryloyloxyethyl trimellitic acid, 4-(meth)acryloyloxybutyloxycarbonylphthalic acid, 4-(meth)acryloyloxyhexyloxycarbonylphthalic acid, 4-(meth)acryloyloxyoctyloxycarbonylphthalic acid, and 4-(meth)acryloyloxydecyloxycarbonylphthalic acid, and acid anhydrides thereof, 5-(meth)acryloylamino pentylcarboxylic acid, 6-(meth)acryloyloxy-1,1-hexanedicarboxylic acid, 8-(meth)acryloyloxy-1,1-octanedicarboxylic acid, 10-(meth)acryloyloxy-1,1-decanedicarboxylic acid, and 11-(meth)acryloyloxy-1,1-undecanedicarboxylic acid, and hydrochlorides, alkali metal salts, ammonium salts, and amine salts thereof.

Examples of the polymerizable monomer having a sulfonic acid group include 2-(meth)acrylamido-2-methylpropanesulfonic acid and 2-sulfoethyl (meth)acrylate, and hydrochlorides, alkali metal salts, ammonium salts, and amine salts thereof.

Among the polymerizable monomers (A) having an acidic group, a polymerizable monomer having a phosphoric acid group, a polymerizable monomer having a pyrophosphoric acid group, and a polymerizable monomer having a carboxylic acid group are preferred since the further excellent adhesion to the tooth substance is exhibited thereby, and a polymerizable monomer having a phosphoric acid group and a polymerizable monomer having a carboxylic acid group are particularly preferred. Among the above, a (meth)acrylate based monofunctional polymerizable monomer having in the molecule thereof an alkyl group having 6 to 20 carbon atoms or an alkylene group having 6 to 20 carbon atoms as a main chain, and having a phosphoric acid group, or a (meth)acrylate based monofunctional polymerizable monomer having in the molecule thereof an alkyl group having 6 to 20 carbon atoms or an alkylene group having 6 to 20 carbon atoms as a main chain, and having a carboxylic acid group are more preferred, and a (meth)acrylate based monofunctional polymerizable monomer having in the molecule thereof an alkylene group having 8 to 12 carbon atoms as a main chain, and having a phosphoric acid group is further preferred. 10-Methacryloyloxydecyl dihydrogen phosphate, 4-(meth) acryloyloxyethyl trimellitic acid, and 4-(meth)acryloyloxyethyl trimellitic anhydride are preferred, and 10-methacryloyloxydecyl dihydrogen phosphate is most preferred.

One kind of the polymerizable monomer (A) having an acidic group may be blended alone, or two or more kinds

thereof may be blended in combination. The content of the polymerizable monomer (A) having an acidic group is not particularly limited, as long as exhibiting the effects of the present invention, and is preferably in a range of 1 to 50 parts by mass, more preferably in a range of 2 to 30 parts by mass, and further preferably in a range of 3 to 25 parts by mass, per 100 parts by mass in total of the polymerizable monomers in the dental adhesive composition of the present invention, from the standpoint of the further excellent adhesion. In the description herein, "100 parts by mass in total of the polymerizable monomers" means the case where the total amount of the polymerizable monomers except for the solvent is assumed to be 100 parts by mass for the bonding material and the pretreatment material, the case where the total amount of the polymerizable monomer contained in the first part and the polymerizable monomer contained in the second part is converted to 100 parts by mass for the dental cement, and the case where the total amount of the polymerizable monomer is assumed to be 100 parts by mass for the dental self-adhesive composite resin.

[Photoredox Catalyst (B)]

The dental adhesive composition of the present invention contains a photoredox catalyst (B) having a singlet excited state reduction potential $E^{S1}_{red}$ of 1.5 V vs SCE or more.

The dental adhesive composition of the present invention is excellent in storage stability under no irradiation of light since the photoredox catalyst (B) is used as a redox catalyst, and furthermore is excellent in adhesion to the tooth substance in photocuring and also maintains the high adhesion to the tooth substance even after storing since the singlet excited state reduction potential $E^{S1}_{red}$ of the photoredox catalyst (B) is 1.5 V vs SCE or more.

The singlet excited state reduction potential $E^{S1}_{red}$ is preferably 1.6 V vs SCE or more, more preferably 1.7 V vs SCE or more, further preferably 1.8 V vs SCE or more, still further preferably 1.9 V vs SCE or more, still more further preferably 2.0 V vs SCE or more, even further preferably 2.2 V vs SCE or more, and even still further preferably 2.5 V vs SCE or more. The upper limit value of the singlet excited state reduction potential $E^{S1}_{red}$ is not particularly limited, and is preferably 3.0 V vs SCE or less from the standpoint of the availability and the like. The singlet excited state reduction potential $E^{S1}_{red}$ is preferably 1.6 to 3.0 V vs SCE, more preferably 1.7 to 3.0 V vs SCE, further preferably 1.8 to 3.0 V vs SCE, still further preferably 1.9 to 3.0 V vs SCE, still more further preferably 2.0 to 3.0 V vs SCE, even further preferably 2.2 to 3.0 V vs SCE, and even still further preferably 2.5 to 3.0 V vs SCE.

The singlet excited state oxidation potential $E^{S1}_{ox}$ of the photoredox catalyst (B) is preferably negative. According to the configuration, it is considered that the catalyst is excited through light irradiation and functions as a strong reducing agent, which largely accelerates the polymerization curing reaction at the adhesion interface, so as to enhance the adhesion to tooth substance.

The singlet excited state oxidation potential $E^{S1}_{ox}$ is preferably -0.1 V vs SCE or less, more preferably -0.5 V vs SCE or less, further preferably -0.8 V vs SCE or less, still further preferably -0.9 V vs SCE or less, and still more further preferably -1.0 V vs SCE or less. The lower limit value of the singlet excited state oxidation potential $E^{S1}_{ox}$ is not particularly limited, and is preferably -3.0 V vs SCE or more from the standpoint of the availability and the like. The singlet excited state oxidation potential $E^{S1}_{ox}$ is preferably -3.0 to -0.1 V vs SCE, more preferably -3.0 to -0.5 V vs SCE, further preferably -3.0 to -0.8 V vs SCE, still further preferably -3.0 to -0.9 V vs SCE, and still more further preferably -3.0 to -1.0 V vs SCE.

The photoredox catalyst (B) is preferably a visible light photoredox catalyst (B). The visible light photoredox catalyst (B) herein means the photoredox catalyst (B) that has an absorption spectrum including a part or the whole of a wavelength band of 400 nm to 800 nm. According to the configuration, the dental adhesive composition of the present invention can be photocured through irradiation of visible light.

The photoredox catalyst (B) preferably has an absorption spectrum including a range of 400 nm or more and 500 nm or less since the absorption spectrum of the photoredox catalyst (B) includes the wavelength of a blue LED, which is the light source used in a dental irradiator, and thereby the photoredox catalyst (B) can be excited with high efficiency, resulting in high reactivity.

The "absorption spectrum including a range of 400 nm or more and 500 nm or less" herein means that the molar absorption coefficient at any wavelength within a range of 400 to 500 nm is 10 or more.

The molar absorption coefficient at any wavelength within the range of 400 to 500 nm is preferably 50 or more, more preferably 80 or more, further preferably 100 or more, still further preferably 1,000 or more, still more further preferably 4,000 or more, and even further preferably 6,000 or more, and is preferably 1,000,000 or less in consideration of the availability and the influence on the color tone of the composition.

The photoredox catalyst (B) used is not particularly limited, and is preferably at least one kind selected from the group consisting of compounds represented by the following formulae (1), (2), and (3):

[Chem. 4]

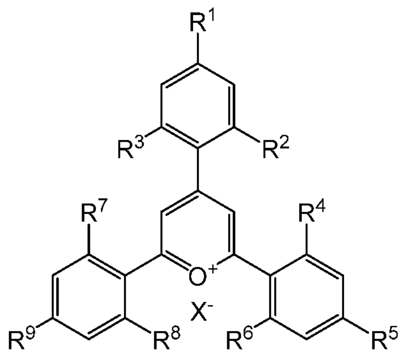

(1)

In the formula (1), $R^1$, $R^2$, $R^3$, $R^4$, $R^5$, $R^6$, $R^7$, $R^8$, and $R^9$ each independently represent a hydrogen atom, an alkyl group having 1 to 10 carbon atoms, an alkoxy group having 1 to 10 carbon atoms, or a halogen atom, and $X^-$ represents $BF_4^-$, $PF_6^-$, $ClO_4^-$, or $HSO_4^-$.

In the formula (1), $R^1$, $R^2$, and $R^3$ each preferably independently represent an alkyl group having 1 to 4 carbon atoms, and more preferably a methyl group, an isopropyl group, or a tert-butyl group.

The compound represented by the formula (1) is more preferably a compound represented by the formula (1A).

In the formula (1A), $R^1$, $R^2$, $R^3$, $R^5$, and $R^9$ have the same meanings as the symbols in the formula (1), respectively.

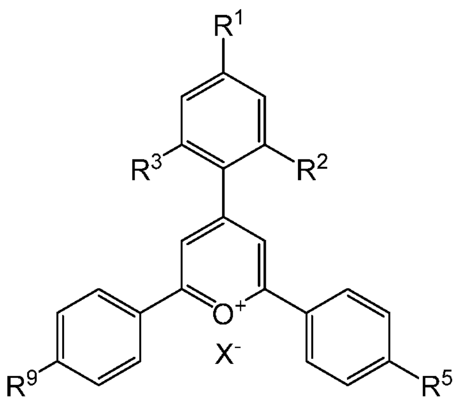

(1A)

[Chem. 5]

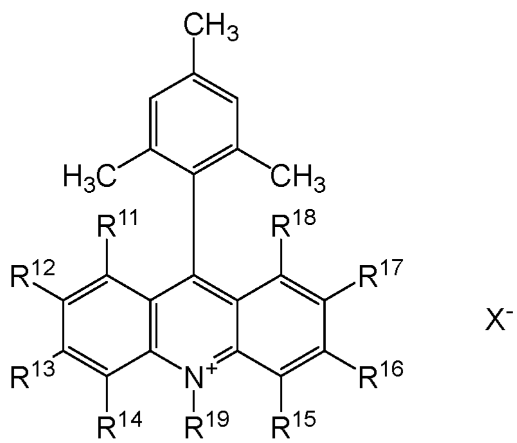

(2)

In the formula (2), $R^{11}$, $R^{13}$, $R^{14}$, $R^{15}$, $R^{16}$, and $R^{18}$ each independently represent a hydrogen atom or an alkyl group having 1 to 10 carbon atoms, $R^{12}$, $R^{17}$, and $R^{19}$ each independently represent a hydrogen atom, an alkyl group having 1 to 10 carbon atoms, or an aryl group having 6 to 30 carbon atoms, and $X^-$ represents $BF_4^-$, $PF_6^-$, $ClO_4^-$, or $HSO_4^-$.

It is preferred that $R^{11}$, $R^{13}$, $R^{14}$, $R^{15}$ , $R^{16}$, and $R^{18}$ represent hydrogen atoms, $R^{12}$ and $R^{17}$ represent hydrogen

atoms, and $R^{19}$ represents an alkyl group having 1 to 4 carbon atoms.

[Chem. 6]

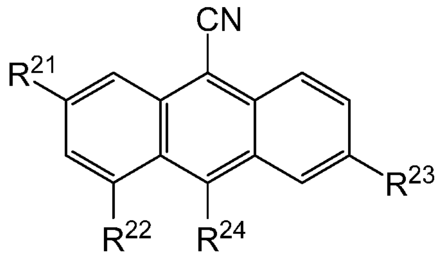

(3)

In the formula (3), $R^{21}$, $R^{22}$, $R^{23}$, and $R^{24}$ each independently represent a hydrogen atom, an alkyl group having 1 to 10 carbon atoms, an alkoxy group having 1 to 10 carbon atoms, a carboxylic acid group having 1 to 10 carbon atoms or an ester thereof, a hydroxy group, a cyano group, or a halogen atom. It is preferred that among $R^{21}$ to $R^{24}$, $R^{21}$, $R^{22}$, and $R^{23}$ represent hydrogen atoms, and $R^{24}$ represents a cyano group or a carboxylate ester group.

Specific examples of the compound represented by the formula (1) include 2,4,6-triphenylpyrylium tetrafluoroborate, 2,4,6-triphenylpyrylium hydrogen sulfate, 2,4,6-tri-p-tolylpyrylium tetrafluoroborate, 2,4,6-tris(4-methoxyphenyl) pyrylium tetrafluoroborate, 2,4,6-tris(4-fluorophenyl)pyrylium tetrafluoroborate, 4-mesityl-2,6-diphenylpyrylium tetrafluoroborate, 4-mesityl-2,6-di-p-tolylpyrylium tetrafluoroborate, 4-(4-butoxyphenyl)-2,6-bis(4-methoxyphenyl)pyrylium tetrafluoroborate, 4-(4-butoxyphenyl)-2,6-diphenylpyrylium tetrafluoroborate, 2,6-bis(4-methoxyphenyl)-4-phenylpyrylium tetrafluoroborate, 4-(4-butoxyphenyl)-2,6-diphenylpyrylium tetrafluoroantimonate, 2,4,6-tri(4-methoxyphenyl)pyrylium perchlorate, 4-(3,4-diethoxyphenyl)-2,6-bis(4-methoxyphenyl)pyrylium tetrafluoroborate, 4-(4-dimethylaminophenyl)-2,6-diphenylpyrylium perchlorate, 2-(4-nitrophenyl)-4,6-bis(4-butylphenyl)pyrylium tetrafluoroborate, and 2-(3,4-dichlorophenyl)-4-(4-methoxyphenyl)-6-phenylpyrylium tetrafluoroborate. Among these, 2,4,6-triphenylpyrylium tetrafluoroborate, 2,4,6-tri-p-tolylpyrylium tetrafluoroborate, 2,4,6-tris(4-methoxyphenyl)pyrylium tetrafluoroborate, 2,4,6-tris(4-fluorophenyl)pyrylium tetrafluoroborate, 4-mesityl-2,6-diphenylpyrylium tetrafluoroborate, and 4-mesityl-2,6-di-p-tolylpyrylium tetrafluoroborate are preferred.

Examples of the compound represented by the formula (2) include 9-mesityl-10-methylacridinium perchlorate, 9-mesityl-10-methylacridinium tetrafluoroborate, 9-mesityl-10-phenylacridinium perchlorate, 9-mesityl-10-phenylacridinium tetrafluoroborate, 9-mesityl-3,6-di-t-butyl-10-phenylacridinium tetrafluoroborate, 9-mesityl-1,3,6,8-tetramethoxy-10-phenylacridinium tetrafluoroborate, and 2,7,10-trimethyl-9-mesitylacridinium perchlorate. Among these, 9-mesityl-10-methylacridinium perchlorate and 9-mesityl-10-methylacridinium tetrafluoroborate are preferred.

Examples of the compound represented by the formula (3) include 9,10-dicyanoanthracene, 9-cyanoanthracene, methyl 9-cyanoanthracene-10-carboxylate, 2,4,9,10-tetracyanoanthracene, and 2,6,9,10-tetracyanoanthracene.

One kind of the photoredox catalyst (B) may be blended alone, or two or more kinds thereof may be blended in combination. The content of the photoredox catalyst (B) is not particularly limited, as long as exhibiting the effects of the present invention, and is preferably 0.001 part by mass or more from the standpoint of the further excellent adhesion, and is preferably 3 parts or less from the standpoint of suppressing coloration and suppressing the shielding of light for sufficiently irradiating the photocatalyst (B) with light, all per 100 parts by mass in total of the polymerizable monomers in the dental adhesive composition of the present invention. From the standpoints, the content of the photoredox catalyst (B) is preferably in a range of 0.001 to 5 parts by mass, more preferably in a range of 0.005 to 3 parts by mass, further preferably in a range of 0.01 to 2 parts by mass, still further preferably in a range of 0.01 to 1.5 parts by mass, still more further preferably in a range of 0.01 to 1 part by mass, and even further preferably in a range of 0.01 to 0.8 part by mass, per 100 parts by mass in total of the polymerizable monomers in the dental adhesive composition of the present invention.

[Polymerizable Monomer (C) having No Acidic Group]

The dental adhesive composition of the present invention may contain or may not contain a polymerizable monomer (C) having no acidic group, and preferably contains the same. The polymerizable monomer (C) having no acidic group is a polymerizable monomer that is formed into a polymer through the progress of radical polymerization reaction with a polymerization initiator system. One kind of the polymerizable monomer (C) having no acidic group may be used alone, or two or more kinds thereof may be used in combination. Preferred examples of the polymerizable monomer (C) having no acidic group include a hydrophilic polymerizable monomer (C-1) and a hydrophobic polymerizable monomer (C-2) shown below.

The hydrophilic polymerizable monomer (C-1) means a polymerizable monomer that has a solubility in water at 25°C of 10% by mass or more. A polymerizable monomer having the solubility that is 30% by mass or more is preferred,

and a polymerizable monomer that can be dissolved in water at any ratio at 25°C is more preferred. The hydrophilic polymerizable monomer (C-1) accelerates the permeation of the components of the dental adhesive composition to the tooth substance, and also permeates the tooth substance by itself and adheres to the organic component (collagen) in the tooth substance.

Examples of the hydrophilic polymerizable monomer (C-1) include a monofunctional (meth)acrylate ester based polymerizable monomer, such as 2-hydroxyethyl acrylate, 2-hydroxyethyl methacrylate (which may be hereinafter abbreviated as "HEMA"), 3-hydroxypropyl (meth)acrylate, 2-hydroxypropyl (meth)acrylate, 1,3-dihydroxypropyl (meth) acrylate, 2,3-dihydroxypropyl (meth)acrylate, and 2-((meth)acryloyloxy)ethyl trimethylammonium chloride; and a bifunctional (meth)acrylate ester based polymerizable monomer, such as polyethylene glycol di(meth)acrylate (number of oxyethylene groups: 9 or more), in which 2-hydroxyethyl (meth)acrylate is preferred.

In the description herein, the "(meth)acryl" means both acryl and methacryl, which is similarly applied to the expressions including "(meth)acryloyl" and "(meth)acrylate".

The hydrophobic polymerizable monomer (C-2) means a polymerizable monomer that has a solubility in water at 25°C of less than 10% by mass. Examples of the hydrophobic polymerizable monomer (C-2) include a monofunctional polymerizable monomer and a bifunctional polymerizable monomer based on an aromatic compound, and a monofunctional polymerizable monomer, a bifunctional polymerizable monomer, and a trifunctional or higher functional polymerizable monomer based on an aliphatic compound. The hydrophobic polymerizable monomer (C-2) enhances the mechanical strength, the handleability, and the like of the dental adhesive composition.

Examples of the aromatic compound based monofunctional polymerizable monomer include benzyl (meth) acrylate, p-cumyl-phenoxyethylene glycol (meth)acrylate, and 2-phenoxybenzyl (meth)acrylate. Among these, benzyl methacrylate, 2-phenoxybenzyl (meth)acrylate and , p-cumyl-phenoxyethylene glycol methacrylate are preferred.

Examples of the aromatic compound based bifunctional polymerizable monomer include an aromatic di(meth) acrylate. Specific examples of the aromatic compound based bifunctional polymerizable monomer include 2,2-bis((meth) acryloyloxyphenyl)propane, 2,2-bis[4-(3-acryloyloxy-2-hydroxypropoxy)phenyl]propane, 2,2-bis[4-(3-methacryloyloxy-2-hydroxypropoxy)phenyl]propane (which may be hereinafter abbreviated as "Bis-GMA"), 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl) propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth) acryloyloxytriethoxyphenyl)propane, 2,2-bis(4-(meth)acryloyloxypropoxyphenyl)propane, 2,2-bis(4-(meth)acryloyloxyisopropoxyphenyl)propane, and 1,4-bis(2-(meth)acryloyloxyethyl) pyromellitate. Among these, 2,2-bis[4-(3-methacryloyloxy-2-hydroxypropoxy)phenyl]propane and 2,2-bis(4-methacryloyloxypolyethoxyphenyl)propane (average addition molar number of ethoxy groups: 2.6) (which may be hereinafter abbreviated as "D-2.6E") are preferred.

Examples of the aliphatic compound based monofunctional polymerizable monomer include methyl (meth) acrylate, ethyl (meth)acrylate, butyl (meth)acrylate, isobornyl (meth)acrylate, stearyl (meth)acrylate, dicyclopentanyl (meth)acrylate, butoxydiethylene glycol (meth)acrylate, methoxypolyethylene glycol (meth)acrylate, and tetrahydrofurfuryl methacrylate. Among these, isobornyl methacrylate and tetrahydrofurfuryl methacrylate (which may be hereinafter abbreviated as "THF-MA") are preferred.

Examples of the aliphatic compound based bifunctional polymerizable monomer include a bifunctional (meth) acrylate ester based polymerizable monomer, such as erythritol di(meth)acrylate, sorbitol di(meth)acrylate, mannitol di(meth)acrylate, pentaerythritol di(meth)acrylate, dipentaerythritol di(meth)acrylate, glycerol di(meth)acrylate (which may be hereinafter abbreviated as "GDMA"), ethylene glycol di(meth)acrylate, diethylene glycol di(meth)acrylate, triethylene glycol di(meth)acrylate (which may be hereinafter abbreviated as "TEGDMA"), propylene glycol di(meth) acrylate, butylene glycol di(meth)acrylate, neopentyl glycol di(meth)acrylate, polyethylene glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 2,2,4-trimethylhexamethylene bis(2-carbamoyloxyethyl) dimethacrylate, and 1,2-bis(3-methacryloyloxy-2-hydroxypropyloxy)ethane; and a (meth)acrylamide based polymerizable monomer, such as N-methacryloyloxy ethyl acrylamide, N-methacryloyloxy propyl acrylamide, N-methacryloyloxy butyl acrylamide, N-(1-ethyl-(2-methacryloyloxy)ethyl) acrylamide, and N-(2-(2-methacryloyloxyethoxy)ethyl) acrylamide. Among these, glycerol dimethacrylate, triethylene glycol di(meth)acrylate, neopentyl glycol dimethacrylate, 2,2,4-trimethylhexamethylene bis(2-carbamoyloxyethyl) dimethacrylate, and 1,2-bis(3-methacryloyloxy-2-hydroxypropyloxy)ethane are preferred.

Examples of the trifunctional or higher functional polymerizable monomer include trimethylolpropane tri(meth) acrylate, trimethylolethane tri(meth)acrylate, trimethylolmethane tri(meth)acrylate, pentaerythritol tri(meth)acrylate, pentaerythritol tetra(meth)acrylate, dipentaerythritol tri(meth)acrylate, dipentaerythritol tetra(meth)acrylate, dipentaerythritol penta(meth)acrylate, N,N-(2,2,4-trimethylhexamethylene)bis[2-(aminocarboxy)propane-1,3-diol] tetramethacrylate, and 1,7-diacryloyloxy-2,2,6,6-tetraacryloyloxymethyl-4-oxaheptane.

In the polymerizable monomer (C) having no acidic group, HEMA, GDMA, Bis-GMA, D-2.6E, TEGDMA, and

THF-MA are more preferred from the standpoint of the adhesion strength and the polymerization curability of the dental adhesive composition of the present invention.

One kind of each of the polymerizable monomers (C) having no acidic group (i.e., the hydrophilic polymerizable monomer (C-1) and the hydrophobic polymerizable monomer (C-2)) may be contained alone, or two or more kinds thereof may be blended in combination.

The content of the polymerizable monomer (C) having no acidic group is not particularly limited, as long as exhibiting the effects of the present invention, and is preferably in a range of 20 to 99 parts by mass, more preferably in a range of 30 to 98 parts by mass, further preferably in a range of 40 to 95 parts by mass, still further preferably in a range of 60 to 95 parts by mass, still more further preferably in a range of 70 to 95 parts by mass, and even further preferably in a range of 80 to 95 parts by mass, per 100 parts by mass in total of the polymerizable monomers in the dental adhesive composition of the present invention, from the standpoint of the high permeability to the tooth substance and the excellent adhesion of the composition, and the sufficient mechanical strength of the cured product.

The content of the hydrophilic polymerizable monomer (C-1) in the total amount of the hydrophilic polymerizable monomer (C-1) and the hydrophobic polymerizable monomer (C-2) is further preferably in a range of 1.0 to 95% by mass, still further preferably in a range of 5.0 to 70% by mass, and still more further preferably in a range of 0.5 to 60% by mass.

The polymerization initiator system will be subsequently described. The dental adhesive composition of the present invention may further contain an organic peroxide (D) and a reducing agent (E) as a polymerization initiator system. With the combination use of the polymerization initiator systems and the use thereof along with the other components, the curability can be set to an appropriate range, the excellent adhesion to the tooth substance and the excellent storage stability can be achieved, and the high adhesion to the tooth substance can be maintained even after storing.

[Organic Peroxide (D)]

The dental adhesive composition of the present invention may contain or may not contain an organic peroxide (D). The dental adhesive composition of the present invention contains the polymerizable monomer (A) having an acidic group and the photoredox catalyst (B), in which the photoredox catalyst (B) can function as an oxidizing agent by itself through excitation even though the organic peroxide (D) is not contained, and thereby the enhanced adhesiveness to the tooth substance and the high storage stability are achieved.

Examples of the organic peroxide (D) include diacyl peroxides, peroxyesters, dialkyl peroxides, peroxyketals, ketone peroxides, and hydroperoxides. Specific examples of the diacyl peroxides include benzoyl peroxide, 2,4-dichlorobenzoyl peroxide, and m-toluoyl peroxide. Specific examples of the peroxyesters include tert-butyl peroxybenzoate, bis(tert-butyl peroxy)isophthalate, 2,5-dimethyl-2,5-bis(benzoylperoxy)hexane, tert-butyl peroxy-2-ethylhexanoate, and tert-butyl peroxyisopropyl carbonate. Specific examples of the dialkyl peroxides include dicumyl peroxide, di-tert-butyl peroxide, and lauroyl peroxide. Specific examples of the peroxyketals include 1,1-bis(tert-butylperoxy)-3,3,5-trimethylcyclohexane, 1,1-bis(tert-butylperoxy)cyclohexane, and 1,1-bis(tert-hexylperoxy)cyclohexane. Specific examples of the ketone peroxides include methyl ethyl ketone peroxide, cyclohexanone peroxide, and methyl acetoacetate peroxide. Specific examples of the hydroperoxides include tert-butyl hydroperoxide, cumene hydroperoxide, p-diisopropylbenzene hydroperoxide, and 1,1,3,3-tetramethylbutyl hydroperoxide.

In the organic peroxide (D), the hydroperoxides and the peroxyesters are preferred. In the hydroperoxides, tert-butyl hydroperoxide, cumene hydroperoxide, and 1,1,3,3-tetramethylbutyl hydroperoxide are preferably used. In the peroxyesters, tert-butyl peroxybenzoate is preferably used.

One kind of the organic peroxide (D) may be blended alone, or two or more kinds thereof may be blended in combination.

In the case where the dental adhesive composition of the present invention contains the organic peroxide (D), the content of the organic peroxide (D) is preferably in a range of 0.01 to 10 parts by mass, more preferably in a range of 0.1 to 5 parts by mass, and further preferably in a range of 0.5 to 3 parts by mass, per 100 parts by mass in total of the polymerizable monomer in the dental adhesive composition of the present invention, from the standpoint of the curability, the mechanical strength of the cured product, the adhesion to the tooth substance, and the storage stability.

[Reducing Agent (E)]

The dental adhesive composition of the present invention may contain or may not contain a reducing agent (E). The dental adhesive composition of the present invention contains the polymerizable monomer (A) having an acidic group and the photoredox catalyst (B), in which the photoredox catalyst (B) can function as a reducing agent by itself through excitation, and thereby the enhanced adhesion to the tooth substance and the high storage stability are achieved.

Examples of the reducing agent (E) include a thiourea compound, sulfinic acid and a salt thereof, a sulfite salt, a bisulfite salt, a borate compound, barbituric acid and a derivative thereof, and an ascorbic acid derivative.

Examples of the thiourea compound include thiourea, methylthiourea, ethylthiourea, ethylenethiourea, N,N'-dimethylthiourea, N,N'-diethylthiourea, N,N'-di-n-propylthiourea, N,N'-dicyclohexylthiourea, trimethylthiourea, triethylthiourea, tri-n-propylthiourea, tricyclohexylthiourea, tetramethylthiourea, tetraethylthiourea, tetra-n-propylthiourea, tetracyclohexylthiourea, 1-(2-pyridyl)-2-thiourea, and 4,4-dimethylethylenethiourea. One kind of the thiourea compound may be used alone, or two or more kinds thereof may be used in combination.

Examples of the sulfinic acid and a salt thereof include p-toluenesulfinic acid, sodium p-toluenesulfinate, potassium p-toluenesulfinate, lithium p-toluenesulfinate, calcium p-toluenesulfinate, benzenesulfinic acid, sodium benzenesulfinate, potassium benzenesulfinate, lithium benzenesulfinate, calcium benzenesulfinate, 2,4,6-trimethylbenzenesulfinic acid, sodium 2,4,6-trimethylbenzenesulfinate, potassium 2,4,6-trimethylbenzenesulfinate, lithium 2,4,6-trimethylbenzenesulfinate, calcium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, lithium 2,4,6-triethylbenzenesulfinate, calcium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, lithium 2,4,6-triisopropylbenzenesulfinate, and calcium 2,4,6-triisopropylbenzenesulfinate. Among these, sodium benzenesulfinate, sodium p-toluenesulfinate, 2,4,6-triisopropylbenzenesulfinic acid, and sodium 2,4,6-triisopropylbenzenesulfinate are preferred. One kind of the sulfinic acid and a salt thereof may be used alone, or two or more kinds thereof may be used in combination.

Examples of the sulfite salt include sodium sulfite, potassium sulfite, calcium sulfite, and ammonium sulfite. Examples of the bisulfite salt include sodium bisulfite and potassium bisulfite. Examples of the borate compound include an aryl borate compound having 1 to 4 aryl groups in one molecule (such as tetraphenylboron and tetrakis(p-chlorophenyl)boron) and a salt thereof. Examples of the barbituric acid and a derivative thereof include barbituric acid, 5-butylbarbituric acid, 1,3,5-tributylbarbituric acid, 1-cyclohexyl-5-ethylbarbituric acid, 1-benzyl-5-phenylbarbituric acid, and salts thereof.

Examples of the ascorbic acid derivative include a salt, an ester, and an ether of ascorbic acid. Among these, a salt and an ester of ascorbic acid is preferred.

Examples of the salt of ascorbic acid include sodium L-ascorbate, calcium L-ascorbate, potassium ascorbate, and stereoisomers thereof (such as sodium isoascorbate). Among these, sodium L-ascorbate is preferred.

Examples of the ester of ascorbic acid include a compound formed by reacting one or more hydroxy group of ascorbic acid with a carboxylic acid. Preferred examples of the carboxylic acid include a fatty acid, such as a saturated fatty acid or unsaturated fatty acid having 6 to 30 carbon atoms, such as caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, $\alpha$-linolenic acid, arachidonic acid, eicosapentaenoic acid, erucic acid, and docosahexaenoic acid. The number of carbon atoms of the fatty acid is preferably 10 to 28, more preferably 12 to 26, and further preferably 14 to 24. Among these, an ester of stearic acid and ascorbic acid and an ester of palmitic acid and ascorbic acid (ascorbyl palmitate) are preferably used.

Examples of the ether of ascorbic acid include ascorbic acid ethyl ether and ascorbic acid cetyl ether.

One kind of the ascorbic acid compound may be blended alone, or two or more kinds thereof may be blended in combination.

In the case where the dental adhesive composition of the present invention contains the reducing agent (E), the content of the reducing agent (E) is not particularly limited, as long as exhibiting the effects of the present invention, and is preferably in a range of 0.01 to 5.0 parts by mass, more preferably in a range of 0.02 to 3.0 parts by mass, and further preferably in a range of 0.03 to 2.0 parts by mass, per 100 parts by mass in total of the polymerizable monomers in the dental adhesive composition of the present invention, from the standpoint of the high permeability to the tooth substance and the excellent adhesions of the composition, and the sufficient mechanical strength of the cured product.

From the standpoint of preventing the organic peroxide (D) and the reducing agent (E) from being reacted with each other in storing, the dental adhesive composition may have an embodiment that contains the organic peroxide (D) but does not contain the reducing agent (E), or may have an embodiment that does not contain the organic peroxide (D) but contains the reducing agent (E).

Specifically, in the case where the dental adhesive composition is a one-component type (one-liquid type or one-paste type), the one-component type dental adhesive composition may have an embodiment that contains the organic peroxide (D) but does not contain the reducing agent (E), or may have an embodiment that does not contain the organic peroxide (D) but contains the reducing agent (E).

In the case where the dental adhesive composition is a two-component type (two-liquid type or two-paste type), the components each independently may have an embodiment that contains the organic peroxide (D) but does not contain the reducing agent (E), or may have an embodiment that does not contain the organic peroxide (D) but contains the reducing agent (E). For example, in the case where the dental adhesive composition is a two-component type, an embodiment may be used in which one of the components contains the organic peroxide (D) but does not contain the reducing agent (E), and the other thereof does not contain the organic peroxide (D) but contains the reducing agent (E). In the case where the dental adhesive composition is a two-component type, an embodiment may be used in which one of the components contains the organic peroxide (D) but does not contain the reducing agent (E), and the other thereof does not

contain the organic peroxide (D) and does not contain the reducing agent (E). In the case where the dental adhesive composition is a two-component type, an embodiment may be used in which one of the components does not contain the organic peroxide (D) but contains the reducing agent (E), and the other thereof does not contain the organic peroxide (D) and does not contain the reducing agent (E).

[Phosphine Compound (F)]

The dental adhesive composition of the present invention may contain or may not contain a phosphine compound (F) having an electron-withdrawing group. It is considered that the phosphine compound (F) having an electron-withdrawing group has a function of preventing polymerization inhibition due to oxygen and promoting the polymerization, and functions as a part of the chemical polymerization initiator system.

Specific examples of the compound (F) include a compound represented by the following formula (4). One kind of the compound (F) may be used alone, or two or more kinds thereof may be used in combination.

[Chem. 7]

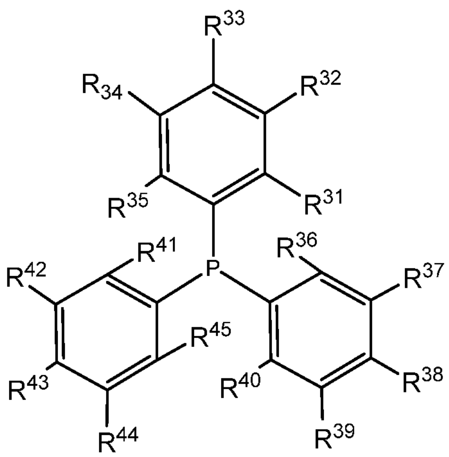

(4)

In the formula (4), $R^{31}$ to $R^{45}$ each independently represent a hydrogen atom, a halogen atom, a polar group, an alkyl group that may have or may not have a substituent, or an alkoxy group that may have or may not have a substituent, in which one or more of $R^{31}$, $R^{33}$, $R^{35}$, $R^{36}$, $R^{38}$, $R^{40}$, $R^{41}$, $R^{43}$, and $R^{45}$ represents at least one electron-withdrawing group selected from the group consisting of a halogen atom, a haloalkyl group, a substituted or unsubstituted acyl group, a substituted or unsubstituted ester group, a substituted or unsubstituted amide group, a substituted or unsubstituted sulfonyl group, and a cyano group.

The alkyl group that may have or may not have a substituent represented by $R^{31}$ to $R^{45}$ may be either linear or branched. The number of carbon atoms of the alkyl group represented by $R^{31}$ to $R^{45}$ is not particularly limited, and is preferably 1 to 12, more preferably 1 to 6, further preferably 1 to 4, and particularly preferably 1 to 3. Examples of the alkyl group represented by $R^{31}$ to $R^{45}$ include a methyl group, an ethyl group, a n-propyl group, an isopropyl group, an n-butyl group, an isobutyl group, a sec-butyl group, a tert-butyl group, a n-pentyl group, an isopentyl group, a sec-pentyl group, a neopentyl group, a n-hexyl group, an isohexyl group, an n-heptyl group, a n-octyl group, a n-nonyl group, a n-decyl group, a n-undecyl group, and a n-dodecyl group. The alkyl group represented by $R^{31}$ to $R^{45}$ may be unsubstituted. Examples of the substituent of the alkyl group represented by $R^{31}$ to $R^{45}$ include a halogen atom, a hydroxy group, an alkoxy group having 1 to 6 carbon atoms, a dialkylamino group and an amino group each having alkyl groups having 1 to 6 carbon atoms,.

Examples of the halogen atom represented by $R^{31}$ to $R^{45}$ include a fluorine atom, a chlorine atom, a bromine atom, and an iodine atom.

Examples of the polar group represented by $R^{31}$ to $R^{45}$ include an acid anhydride group, a carboxylic acid group, a carboxylate ester group, a carboxylic acid chloride group, a carboxylic amide group, a carboxylate salt group, a sulfonic acid group, a sulfonate ester group, a sulfonyl chloride group, a sulfonamide group, a sulfonate salt group, an aldehyde group, an epoxy group, a cyano group, an amino group, a monoalkyl-substituted amino group, a dialkyl-substituted amino group, an imide group, and an oxazoline group, and from the standpoint of the curability and the mechanical strength of the cured product, a carboxylic acid group, a carboxylate ester group, a carboxylic acid chloride group, a carboxylic amide group, a carboxylate salt group, a sulfonic acid group, a sulfonate ester group, a sulfonyl chloride group, a sulfonamide group, a sulfonate salt group, and an aldehyde group are preferred, a carboxylic acid group, a carboxylate ester group, a carboxylic acid chloride group, a carboxylate salt group, a sulfonic acid group, a sulfonate ester group, a sulfonyl chloride group, a sulfonate salt group, and an aldehyde group are more preferred, and a carboxylic acid group, a carboxylate ester

group, a carboxylic acid chloride group, a carboxylate salt group, a sulfonic acid group, a sulfonate ester group, a sulfonyl chloride group, and a sulfonate salt group are further preferred. Examples of the salt of the carboxylate salt group and the sulfonate salt group include a salt of an alkali metal, such as lithium, sodium, and potassium, and a salt of an alkaline earth metal, such as magnesium, calcium, strontium, barium, and radium. In the case where $R^{31}$ to $R^{45}$ represent a polar group, the number of the polar group is preferably 1 to 9, more preferably 1 to 5, and further preferably 1 to 3. In the case where $R^{31}$ to $R^{45}$ represent an alkyl group having a substituent, specific examples thereof include a trifluoromethyl group.

The alkoxy group that may have or may not have a substituent represented by $R^{31}$ to $R^{45}$ may be either linear or branched. The number of carbon atoms of the alkoxy group represented by $R^{31}$ to $R^{45}$ is not particularly limited, and is preferably 1 to 12, more preferably 1 to 6, further preferably 1 to 4, and particularly preferably 1 to 3. Examples of the alkoxy group represented by $R^{31}$ to $R^{45}$ include a methoxy group, an ethoxy group, a n-propoxy group, an isopropoxy group, a n-butoxy group, a sec-butoxy group, a tert-butoxy group, a n-pentyloxy group, an isopentyloxy group, a sec-pentyloxy group, a tert-pentyloxy group, a neopentyloxy group, a n-hexyloxy group, an isohexyloxy group, a sec-hexyloxy group, a tert-hexyloxy group, and a neohexyloxy group. Examples of the substituent of the alkoxy group represented by $R^{31}$ to $R^{45}$ include the same ones as the substituent of the alkyl group represented by $R^{31}$ to $R^{45}$.

$R^{31}$ to $R^{45}$ may be the same as or different from each other. For example, a part of $R^{31}$ to $R^{45}$ may represent the same hydrogen atoms, the same alkyl groups, or the same alkoxy groups.

Examples of the compound represented by the formula (4) include a phosphine compound, such as (2-fluorophenyl)diphenylphosphine, (2-chlorophenyl)diphenylphosphine, (2-bromophenyl)diphenylphosphine, (pentafluorophenyl)diphenylphosphine, bis(pentafluorophenyl)phenylphosphine (which may be hereinafter referred to as "BPFPP"), tris(pentafluorophenyl)phosphine (which may be hereinafter referred to as "TPFPP"), tris(4-fluorophenyl)phosphine (which may be hereinafter referred to as "TFPP"), tris(4-chlorophenyl)phosphine, tris(4-bromophenyl)phosphine, tris(4-trifluoromethylphenyl)phosphine, tris(4-carboxyphenyl)phosphine, sodium diphenylphosphinobenzene-3-sulfonate, and trisodium triphenylphosphine-3,3',3"-trisulfonate.

In the case where the dental adhesive composition of the present invention contains the compound (F), the content of the compound (F) is preferably in a range of 0.005 to 10 parts by mass, more preferably in a range of 0.01 to 5 parts by mass, and further preferably in a range of 0.05 to 3 parts by mass, per 100 parts by mass in total of the polymerizable monomers in the dental adhesive composition of the present invention, from the standpoint of the curability, the mechanical strength of the cured product, and the adhesion to the tooth substance.

[Photopolymerization Initiator (G)]

The dental adhesive composition of the present invention may contain or may not contain an ordinary photopolymerization initiator (G). From the standpoint of the strong adhesion and the storage stability, the photopolymerization initiator (G) is preferably contained. Examples of the photopolymerization initiator (G) include α-diketones, ketals, thioxanthones, (bis)acylphosphine oxides, and α-aminoacetophenones.

Examples of the α-diketones include dl-camphorquinone (trivial name: CQ), benzil, and 2,3-pentandione.

Examples of the ketals include benzyl dimethyl ketal and benzyl diethyl ketal.

Examples of the thioxanthones include 2-chlorothioxanthone and 2,4-diethylthioxanthone.

In the (bis)acylphosphine oxides, examples of the acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,3,5,6-tetramethylbenzoyldiphenylphosphine oxide, 2,4,6-trimethylbenzoylmethoxyphenylphosphine oxide, 2,4,6-trimethylbenzoylethoxyphenylphosphine oxide, benzoylbis(2,6-dimethylphenyl)phosphine oxide, the water-soluble acylphosphine oxide compounds described in JP 3-57916 B, and salts thereof (such as a sodium salt, a potassium salt, and an ammonium salt). Examples of the bisacylphosphine oxides include bis(2,4,6-trimethylbenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, dibenzoylphenylphosphine oxide, bis(2,6-dimethoxybenzoyl)phenylphosphine oxide, tris(2,4-dimethylbenzoyl) phosphine oxide, tris(2-methoxybenzoyl)phosphine oxide, and salts thereof (such as a sodium salt, a potassium salt, and an ammonium salt). Among these (bis)acylphosphine oxides, 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and 2,4,6-trimethylbenzoylphenylphosphine oxide sodium salt are preferred.

Examples of the α-aminoacetophenones include 2-benzyl-2-(dimethylamino)-1-(4-morpholinophenyl)-1-butanone, 2-benzyl-2-(diethylamino)-1-(4-morpholinophenyl)-1-butanone, 2-benzyl-2-(dimethylamino)-1-(4-morpholinophenyl)-1-propanone, 2-benzyl-2-(diethylamino)-1-(4-morpholinophenyl)-1-propanone, 2-benzyl-2-(dimethylamino)-1-(4-morpholinophenyl)-1-pentanone, and 2-benzyl-2-(diethylamino)-1-(4-morpholinophenyl)-1-pentanone.

One kind of the photopolymerization initiator (G) may be blended alone, or two or more kinds thereof may be blended in combination.

In the case where the dental adhesive composition of the present invention contains the photopolymerization initiator (G), the content of the photopolymerization initiator (G) is not particularly limited, and is preferably in a range of 0.001 to 10 parts by mass, more preferably in a range of 0.005 to 5 parts by mass, and further preferably in a range of 0.01 to 3 parts by mass, per 100 parts by mass in total of the polymerizable monomers in the dental adhesive composition of the present invention, from the standpoint of the curability and the like of the resulting dental adhesive composition.

[Polymerization Accelerator]

For enhancing the photocurability, a polymerization accelerator for a photopolymerization initiator may be used in combination with or may not be used in combination with the photopolymerization initiator, and is preferably used in combination therewith. Examples of the polymerization accelerator for a photopolymerization initiator include tertiary amines, aldehydes, a thiol compound, and a triazine compound substituted by a trihalomethyl group.

Examples of the tertiary amines include N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, N,N-bis(2-hydroxyethyl)-3,5-dimethylaniline, N,N-di(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, N,N-bis(2-hydroxyethyl)-3,5-di-t-butylaniline, n-butoxyethyl 4-(N,N-dimethylamino)benzoate, 2-(methacryloyloxy)ethyl 4-(N,N-dimethylamino)benzoate, ethyl 4-(N,N-dimethylamino)benzoate, butyl 4-(N,N-dimethylamino)benzoate, N-methyldiethanolamine, 4-(N,N-dimethylamino)benzophenone, trimethylamine, triethylamine, N-methyldiethanolamine, N-ethyldiethanolamine, N-n-butyldiethanolamine, N-lauryldiethanolamine, triethanolamine, 2-(dimethylamino)ethyl (meth)acrylate, N-methyldiethanolamine di(meth)acrylate, N-ethyldiethanolamine di(meth)acrylate, triethanolamine mono(meth)acrylate, triethanolamine di(meth)acrylate, and triethanolamine tri(meth)acrylate. Examples of the aldehydes include terephthalaldehyde and a benzaldehyde derivative. Examples of the benzaldehyde derivative include dimethylaminobenzaldehyde, p-methoxybenzaldehyde, p-ethoxybenzaldehyde, and p-n-octyloxybenzaldehyde. Examples of the thiol compound include 3-mercaptopropyltrimethoxysilane, 2-mercaptobenzoxazole, decanethiol, and thiobenzoic acid. The triazine compound substituted by a trihalomethyl group used may be any of the known compounds with no particular limitation, as long as being an s-triazine compound having at least one trihalomethyl group, such as a trichloromethyl group and a tribromomethyl group.

One kind of the polymerization accelerator for a photopolymerization initiator may be used alone, or two or more kinds thereof may be used in combination.

In the case where the dental adhesive composition of the present invention contains the polymerization accelerator for a photopolymerization initiator, the content of the polymerization accelerator for a photopolymerization initiator is not particularly limited, and is preferably 0.001 to 10 parts by mass, more preferably 0.005 to 5 parts by mass, and further preferably 0.01 to 3 parts by mass, per 100 parts by mass in total of the polymerizable monomers in the dental adhesive composition of the present invention, from the standpoint of the curability and the like of the resulting dental adhesive composition.

[Filler (H)]

The dental adhesive composition of the present invention may contain or may not contain a filler (H) for achieving the sufficient handleability of the composition and the sufficient mechanical strength of the cured product.

Any filler may be used as the filler (H) unless the effects of the present invention are impaired, and examples thereof include an inorganic filler, an organic filler, and a composite filler of an inorganic filler and an organic filler. One kind of the filler (H) may be blended alone, or two or more kinds thereof may be blended in combination. The average particle diameter of the filler (H) is preferably 0.001 to 10 $\mu$m, and the average particle diameter is more preferably 0.001 to 5 $\mu$m.

Examples of the inorganic filler include silica; a mineral containing silica as a base material, such as kaolin, clay, and mica; ceramics and a glass material containing silica as a base material and containing $Al_2O_3$, $B_2O_3$, $TiO_2$, $ZrO_2$, BaO, $La_2O_3$, SrO, ZnO, CaO, $P_2O_5$, $Li_2O$, $Na_2O$, and the like. Examples of the glass material include lithium borosilicate glass, borosilicate glass, bioglass, lanthanum glass, barium glass, strontium glass, soda glass, zinc glass, and fluoroaluminosilicate glass. Preferred examples of the inorganic filler also include crystalline quartz, hydroxyapatite, alumina, titanium oxide, yttrium oxide, zirconia, barium sulfate, aluminum hydroxide, sodium fluoride, potassium fluoride, sodium monofluorophosphate, lithium fluoride, and ytterbium fluoride. Fine particle silica having an average particle diameter of 0.001 to 0.1 $\mu$m is preferably used from the standpoint of the adhesion and the handleability. Examples of the commercially available product thereof include "Aerosil (registered trade name) OX50", "Aerosil (registered trade name) 50", "Aerosil (registered trade name) 200", "Aerosil (registered trade name) 380", "Aerosil (registered trade name) R972", "Aerosil (registered trade name) 130", and "Aeroxide (registered trade name) Alu C" (trade names, all available from Nippon Aerosil Co., Ltd.). In the present invention, in the case where the inorganic filler is subjected to a surface treatment

described later, the average particle diameter of the inorganic filler means the average particle diameter before the surface treatment.

Examples of the organic filler include a polymer of polymethyl methacrylate, polyethyl methacrylate, or a polyfunctional methacrylate, polyamide, polystyrene, polyvinyl chloride, chloroprene rubber, nitrile rubber, and styrene-butadiene rubber.

Examples of the composite filler of an inorganic filler and an organic filler include a filler including an organic filler having an inorganic filler dispersed therein, and an inorganic-organic composite filler including an inorganic filler coated with various polymers.

The filler (H) may be subjected to a surface treatment in advance with a known surface treatment agent, such as a silane coupling agent, and then may be used, for enhancing the curability, the mechanical strength, and the handleability. Examples of the surface treatment agent include vinyltrimethoxysilane, vinyltriethoxysilane, vinyltrichlorosilane, vinyltris(β-methoxyethoxy)silane, γ-methacryloyloxypropyltrimethoxysilane, γ-glycidoxypropyltrimethoxysilane, γ-mercaptopropyltrimethoxysilane, and γ-aminopropyltriethoxysilane.

The average particle diameter (average primary particle diameter) can be obtained through the laser diffraction-scattering method or observation of the particles with an electron microscope. Specifically, the laser diffraction-scattering method is convenient for the particle diameter measurement of particles of 0.1 μm or more, and the observation with an electron microscope is convenient for the particle diameter measurement of ultrafine particles of less than 0.1 μm. The diameter of 0.1 μm is a value that is measured through the laser diffraction-scattering method. In the laser diffraction-scattering method, for example, the measurement may be performed on the volume basis with a laser diffraction particle diameter distribution analyzer (SALD-2300, available from Shimadzu Corporation) using a 0.2% sodium hexametaphosphate aqueous solution as a dispersion medium. In the observation with an electron microscope, a scanning electron microscope (such as SU3800 and S-4000, available from Hitachi HighTechnologies Corporation) may be used. In the observation with an electron microscope, the average particle diameter can be obtained in such a manner that an electron micrograph of the particles is obtained, and the particle diameters of the particles (200 or more) observed in the unit visual field of the micrograph are measured with an image analyzing particle size distribution analysis software (Mac-View (available from Mountech Co., Ltd.). In this case, the particle diameter is obtained as an arithmetic average value of the maximum length and the minimum length of the particle, and the average primary particle diameter is calculated from the number of the particles and the particle diameters thereof.

In the case where the dental adhesive composition of the present invention contains the filler (H), the content of the filler (H) is not particularly limited, as long as exhibiting the effects of the present invention, and is preferably in a range of 1 to 300 parts by mass, and more preferably in a range of 3 to 250 parts by mass, per 100 parts by mass in total of the polymerizable monomers in the dental adhesive composition of the present invention. Within the range, the sufficient mechanical strength of the cured product and the ease of handling can be obtained.

[Transition Metal Compound]

The dental adhesive composition of the present invention may contain or may not contain a transition metal compound. The transition metal compound is not particularly limited, and examples thereof include a copper compound and a vanadium compound.

Examples of the copper compound include a copper(II) carboxylate, a copper(II) β-diketonate, a copper(II) β-ketoester, a copper alkoxide, a copper dithiocarbamate, and a salt of copper and an inorganic acid. Examples of the copper(II) carboxylate include copper(II) citrate, copper(II) acetate, copper(II) phthalate, copper(II) tartrate, copper(II) oleate, copper(II) octanoate, copper(II) octenoate, copper(II) naphthenate, copper(II) methacrylate, and copper(II) 4-cyclohexylbutanoate. Examples of the copper(II) β-diketonate include copper(II) acetylacetonate, copper(II) trifluoroacetylacetonate, copper(II) hexafluoroacetylacetonate, copper(II) 2,2,6,6-tetramethyl-3,5-heptanedionate, and copper(II) benzoylacetonate. Examples of the copper(II) β-ketoester include copper(II) ethyl acetoacetate. Examples of the copper alkoxide include copper(II) methoxide, copper(II) ethoxide, copper(II) isopropoxide, copper(II) 2-(2-butoxyethoxy)ethoxide, and copper(II) 2-(2-methoxyethoxy)ethoxide. Examples of the copper dithiocarbamate include copper(II) dimethyldithiocarbamate. Examples of the salt of copper and an inorganic acid include copper(II) nitrate, copper(II) bromide, and copper(II) chloride. One kind of these materials may be used alone, or two or more kinds thereof may be used in combination. Among these, a copper(II) carboxylate, a copper(II) β-diketonate, and a copper(II) β-ketoester are preferred, and copper(II) acetate and copper(II) acetylacetonate are more preferred, from the standpoint of the solubility in the polymerizable monomer and the reactivity.

The vanadium compound is preferably a IV-valent and/or V-valent vanadium compound. Examples of the IV-valent and/or V-valent vanadium compound include divanadium(IV) tetraoxide, vanadyl(IV) acetylacetonate, vanadium(IV) oxide stearate, oxovanadium(IV) oxalate, vanadyl(IV) sulfate, vanadium naphthenate, vanadium benzoylacetonate, oxovanadium(IV) bis(maltolate), vanadium(IV) oxobis(1-phenyl-1,3-butanedionate), vanadium(V) pentoxide, vanadium(V) oxytriisopropoxide, sodium metavanadate(V), and ammonium metavanadate(V). Among these, vanadium

acetylacetonate, vanadyl(IV) acetylacetonate, and oxovanadium(IV) bis(maltolate) are preferred, and vanadyl(IV) acetylacetonate and oxovanadium(IV) bis(maltolate) are more preferred, from the standpoint of the adhesion and the like. One kind of the vanadium compound may be used alone, or two or more kinds thereof may be used in combination.

The combination use of the transition metal compound with the other components can enhance the curability of the composition.

In the case where the dental adhesive composition of the present invention contains the transition metal compound, the content of the transition metal compound is preferably in a range of 0.0001 to 1 part by mass, more preferably in a range of 0.0005 to 0.5 part by mass, and further preferably in a range of 0.0008 to 0.2 part by mass, per 100 parts by mass in total of the polymerizable monomers in the dental adhesive composition of the present invention, from the standpoint of the curability, the mechanical strength, and the adhesion to the tooth substance.

[Fluoride Ion Releasing Substance]

The dental adhesive composition of the present invention may further contain or may not further contain a fluoride ion releasing substance. A fluoride ion releasing substance contained can provide the dental adhesive composition that can impart acid resistance to the tooth substance. Examples of the fluoride ion releasing substance include a fluoride ion releasing polymer, such as a copolymer of methyl methacrylate and methacrylic acid fluoride; a hydrofluoride of an aliphatic or alicyclic primary, secondary, or tertiary amine, such as cetylamine hydrofluoride, cyclohexylamine hydrofluoride, diisobutylamine hydrofluoride, and triethylamine trihydrofluoride; and a metal fluoride compound, such as sodium fluoride, potassium fluoride, sodium monofluorophosphate, lithium fluoride, and ytterbium fluoride. One kind of the fluoride ion releasing substance may be used alone, or two or more kinds thereof may be used in combination.

In the case where the dental adhesive composition of the present invention contains the fluoride ion releasing substance, the content of the fluoride ion releasing substance is preferably in a range of 0.01 to 30, more preferably in a range of 0.02 to 20 parts by mass, and further preferably in a range of 0.05 to 15 parts by mass, per 100 parts by mass in total of the polymerizable monomer in the dental adhesive composition of the present invention.

[pH Modifier]

The dental adhesive composition of the present invention may contain or may not contain pH modifier. The pH modifier is used for the purpose of modifying the pH and stabilizing the pH of the dental adhesive composition of the present invention. The pH modifier is not particularly limited, as long as exhibiting the effects of the present invention, and a tertiary aliphatic amine is preferably used. Examples of the tertiary aliphatic amine used in the present invention include N-methyldiethanolamine, N-ethyldiethanolamine, N-n-butyldiethanolamine, N-lauryldiethanolamine, 2-(dimethylamino) ethyl (meth)acrylate, N-methyldiethanolamine di(meth)acrylate, N-ethyldiethanolamine di(meth)acrylate, triethanolamine mono(meth)acrylate, triethanolamine di(meth)acrylate, triethanolamine tri(meth)acrylate, triethanolamine, trimethylamine, triethylamine, and tributylamine. Among these, N-methyldiethanolamine and triethanolamine are preferred. Triethanolamine and 2-(dimethylamino)ethyl methacrylate are preferably used. One kind of the pH modifier may be used alone, or two or more kinds thereof may be used in combination.

In the case where the dental adhesive composition of the present invention contains the pH modifier, the content of the pH modifier is preferably in a range of 0.1 to 5.0 parts by mass, more preferably in a range of 0.5 to 3.0 parts by mass, and further preferably in a range of 1.0 to 2.0 parts by mass, per 100 parts by mass in total of the polymerizable monomer in the dental adhesive composition of the present invention.

[Solvent]

The dental adhesive composition of the present invention may contain or may not contain a solvent in such a range that does not impair the effects of the present invention. One kind thereof may be blended alone, or two or more kinds thereof may be used in combination.

Examples of the solvent include an aqueous solvent, such as water and an alcohol (e.g., ethanol), and an organic solvent.

In the case where the dental adhesive composition of the present invention contains the solvent, the content of the solvent is preferably in a range of 10 to 80 parts by mass, more preferably in a range of 20 to 60 parts by mass, and further preferably in a range of 30 to 50 parts by mass, per 100 parts by mass in total of the polymerizable monomer in the dental adhesive composition of the present invention.

[Additional Additives]

The dental adhesive composition of the present invention may contain or may not contain additives, such as a

polymerization inhibitor, an ultraviolet ray absorbent, a thickener, a colorant, an antibacterial agent, and a perfume, in such a range that does not impair the effects of the present invention. One kind thereof may be blended alone, or two or more kinds thereof may be used in combination.

Examples of the polymerization inhibitor include hydroquinone, hydroquinone monomethyl ether, dibutylhydroquinone, dibutylhydroquinone monomethyl ether, tert-butylcatechol, 2-tert-butyl-4,6-dimethylphenol, 2,6-di-tert-butylphenol, and 2,6-di-tert-butyl-4-methylphenol.

In the case where the dental adhesive composition of the present invention contains the additional additive, the content of the additional additive is preferably in a range of 0.01 to 15.0 parts by mass, more preferably in a range of 0.01 to 10.0 parts by mass, further preferably in a range of 0.01 to 3.0 parts by mass, still further preferably in a range of 0.05 to 2.0 parts by mass, and still more further preferably in a range of 0.05 to 0.2 part by mass, per 100 parts by mass in total of the polymerizable monomer in the dental adhesive composition of the present invention.

The content of the polymerizable monomers is preferably in a range of 80 to 99.9 parts by mass, more preferably in a range of 85 to 99.5 parts by mass, and further preferably in a range of 89 to 99.0 parts by mass, per 100 parts by mass in total of the components of the dental adhesive composition of the present invention except for the solvent and the filler.

[Production Method of Dental Adhesive Composition]

The dental adhesive composition of the present invention may be produced according to an ordinary method corresponding to the kinds and the amounts of the components described above. The dental adhesive composition of the present invention may be used as an embodiment of one-component type or two-component type. The embodiment of one-component type practiced may be selected from embodiments of a liquid material and an embodiment of a paste, and examples thereof include a one-component type dental bonding material and a one-paste type dental self-adhesive composite resin. The embodiment of two-component type practiced may be appropriately selected from embodiments including a powder material and a liquid material, an embodiment including a paste and a liquid material, an embodiment of two-paste type, and the like, and in a more preferred embodiment from the standpoint of the ease of handling, an embodiment of two-paste type may be used. In the embodiment of two-paste type, it is preferred that the pastes are stored in a state where the pastes are separated from each other, and the two pastes are kneaded immediately before use, and cured through the chemical polymerization proceeding. The paste is generally produced by kneading a liquid component prepared by mixing the components except for the filler (H) with the filler (H) (powder). Examples of the embodiment of two-paste type include dental self-adhesive resin cement.

[Kit]

The dental adhesive composition of the present invention can also be used as a kit. Examples of the kit include a kit including a one-component type dental bonding material containing the photoredox catalyst (B) (i.e., the dental adhesive composition of the present invention), and a dental two-paste type composition, and a kit including a one-component type dental bonding material containing the photoredox catalyst (B) (i.e., the dental adhesive composition of the present invention), and a dental one-paste type composition.

[Applications of Dental Adhesive Composition and Kit]

The dental adhesive composition and the kit of the present invention can be preferably applied to a one-component type dental bonding material, a dental self-adhesive composite resin, and dental self-adhesive resin cement.

The dental adhesive composition and the kit of the present invention can be used as a filling material for dental crown restoration for a missing part of a tooth defeated part, and for adhering a dental prosthesis, such as a crown, an inlay, and a bridge, to the tooth substance.

The present invention encompasses embodiments including various combinations of the aforementioned configurations, within the scope of the technical concept of the present invention, as long as exhibiting the effects of the present invention.

Examples

The present invention will be described in detail with reference to examples and comparative examples below, but the present invention is not limited to the examples. The abbreviated terms and the abbreviated symbols used below are as follows. The compounds and the fillers used in the examples and the comparative examples below are all commercially available products except for the cases where the production method thereof is particularly described.

[Polymerizable Monomer (A) having Acidic Group]

MDP: 10-methacryloyloxydecyl dihydrogen phosphate
GPDM: 1,3-dimethacryloyloxypropyl dihydrogen phosphate (glycerol phosphate dimethacrylate)
4-META: 4-methacryloyloxyethyl trimellitic anhydride

[Photoredox Catalyst (B)]

TPT: 2,4,6-triphenylpyrylium tetrafluoroborate (available from Tokyo Kasei Kogyo Co., Ltd.)
MDPT: 4-mesityl-2,6-diphenylpyrylium tetrafluoroborate (synthesized product)
Acr+Mes: 9-mesityl-10-methylacridinium tetrafluoroborate (available from Sigma-Aldrich Co. LLC)
DCA: 9,10-dicyanoanthracene (available from Sigma-Aldrich Co. LLC)

[Synthesis of MDPT]

Under a nitrogen atmosphere, the following compound 1 (21.0 g, 84.6 mmol, 1.0 eq) and THF (1.68 L) were mixed, to which a 1 M THF solution of 2-mesitylmagnesium bromide (423 mL, 423 mmol, 5.0 eq) was added in a dropwise manner, and the mixture was agitated at room temperature (25°C) overnight, so as to provide a reaction liquid. $NaHCO_3$ aq. was added to the reaction liquid, which was then concentrated under reduced pressure. $CH_2Cl_2$ and $H_2O$ were added to the concentrated residue, and the mixture was then filtered. The filtrate was allowed to stand to separate into an aqueous layer and an organic layer, and the aqueous layer was extracted to $CH_2Cl_2$. After combining the organic layer, the mixture was rinsed with NaCl aq., dried over $Na_2SO_4$, and the filtered and concentrated under reduced pressure. The concentrated residue was dissolved in $Et_2O$ (840 mL), to which a mixed liquid of $HBF_4 \cdot Et_2O$ (16.5 g, 102 mmol, 1.2 eq) and $Et_2O$ (210 mL) was added in a dropwise manner. After agitating under ice cooling for 1 hour, the precipitate was collected through filtration. The filtrate was agitated at room temperature overnight, and the precipitate was again collected through filtration. The solid matters thus collected through filtration were combined and dried under reduced pressure, so as to provide MDPT as a yellow solid matter (19.9 g, yield: 54%).

[Chem. 8]

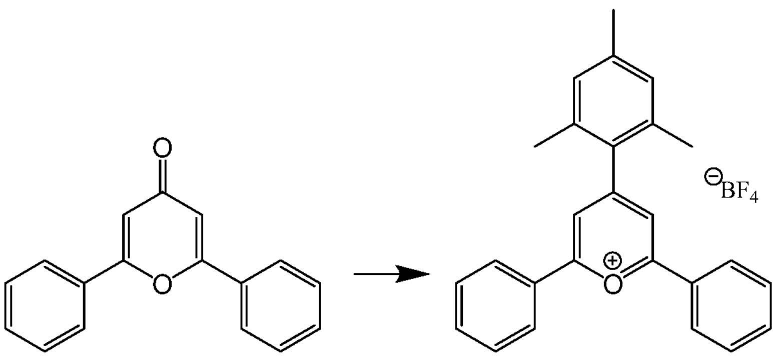

Compound 1

MDPT

[Photoredox Catalyst (B') other than Photoredox Catalyst (B)]

EosinY: 2',4',5',7'-tetrabromofluorescein
Fluorescein

[Maximum Absorption Wavelength ($\lambda_{max}$) and Molar Absorption Coefficient ($\varepsilon$) in 400 nm to 500 nm of Photoredox Catalysts (B) and (B')]

The photoredox catalysts (B) and (B') were measured for the maximum absorption wavelength ($\lambda_{max}$) of the light in a range of 350 to 550 nm in the following manner, and the molar absorption coefficient ($\varepsilon$) in 400 nm to 500 nm was calculated in the following manner.

The photoredox catalyst (B) or (B') was collected at 0.001 mg and dissolved in a solvent in a 50 mL measuring flask to provide a solution. The solvent used was chloroform only for the case where the photoredox catalyst was DCA, and was methanol for all the other photoredox catalysts. The solution was charged in a quartz glass cell (T-1-UV-10, available from Tosoh Quartz Corporation), and measured for the UV/VIS spectrum with a spectrophotometer U-1900 (available from Hitachi HighTechnologies Corporation). An accurate spectrum cannot be obtained with an absorbance of 2 or more at the maximum absorption wavelength, and therefore in the case where the absorbance at the maximum absorption wavelength became 2 or more, the specimen was further diluted to perform the measurement at a concentrate providing an absorbance of 2 or less. The maximum absorption wavelength ($\lambda_{max}$) in a range of 350 nm to 550 nm was obtained from the absorption spectrum measured in the aforementioned manner.

A wavelength at a point near the maximum of light absorption was selected in 400 nm to 500 nm, and from the absorbance (A) at the wavelength, the molar concentration c (mol/L) of the specimen, and the light path length l (cm), the molar absorption coefficient ($\varepsilon$) was calculated by the following expression according to the Lambert-Beer law.

$$A\ (\text{absorbance}) = \varepsilon c l$$

(c: mol/L, l: light path length = 0.993 cm)

The results are shown in Table 1.

[$E^{S1}_{red}$ and $E^{S1}_{ox}$ of Photoredox Catalysts (B) and (B')]

The photoredox catalysts (B) and (B') were measured for the singlet excited state reduction potential $E^{S1}_{red}$ and the singlet excited state oxidation potential $E^{S1}_{ox}$ in the following manner.

A 0.1 M solution of tetrabutylammonium hexafluorophosphate ($TBAPF_6$, available from Sigma-Aldrich Co. LLC) prepared by using anhydrous acetonitrile was charged in an electrochemical cell and deaerated through nitrogen bubbling under application of ultrasonic wave for 10 to 15 minutes. The photoredox catalyst (B) or (B') was added thereto to make a concentration of 1 mM to 45 mM. Thereafter, an electrochemical measurement according to the cyclic voltammetry method was performed with a potentiostat (SP-200, available from BioLogic Science Instruments Ltd.) under condition of a sweep rate of 100 mV/s or less with a working electrode (platinum), a counter electrode (platinum), and a reference electrode (Ag/AgCl, 3 M, NaCl). The resulting value was converted to SCE by subtracting 0.03 V based on the reference electrode (Ag/AgCl, 3 M, NaCl). The oxidation potential $E^{ox}_{1/2}$ and the reduction potential $E^{red}_{1/2}$ in the ground state were obtained in this manner.

The singlet excited state reduction potential ($E^{S1}_{red}$) (V vs SCE) and the singlet excited state oxidation potential ($E^{S1}_{ox}$) (V vs SCE) were obtained by using the aforementioned values and the values of the singlet excitation energy ($E^{S1}_{0.0}$) (literature data*) according to the following expressions.

singlet excited state reduction potential: $E^{S1}_{red} = E^{S1}_{0,0}+E^{red}_{1/2}$
singlet excited state oxidation potential: $E^{S1}_{ox} = E^{OX}_{1/2}-E^{S1}_{0,0}$
*Literature data: $E^{S1}_{0.0}$ values in Chem. Rev. 2016, 116, 10075-10166

The results are shown in Table 1.

[Table 1]

Table 1

| | | $\lambda_{max}$ (nm) | ε (wavelength) (-) | $E^{S1}_{red}$ (V vs SCE) | $E^{S1}_{ox}$ (V vs SCE) |
|---|---|---|---|---|---|
| Photoredox catalyst (B) | TPT | 415 | 21091 (410) | 2.55 | -1.08 |
| | MDPT | 400 | 19323 (400) | 2.62 | -1.15 |
| | $Acr^{+}Mes$ | 425 | 6163 (420) | 2.18 | -0.94 |
| | DCA | 422 | 21627 (425) | 1.99 | -1.08 |
| Photoredox catalyst (B') | EosinY | 520 | 3175 (460) | 1.23 | -1.58 |
| | Fluorescein | 437 | 3292 (450) | 1.25 | -1.55 |

[Polymerizable Monomer (C) having No Acidic Group]

[Hydrophilic Polymerizable Monomer (C-1)]

HEMA: 2-hydroxyethyl methacrylate

[Hydrophobic Polymerizable Monomer (C-2)]

GDMA: glycerol dimethacrylate

Bis-GMA: 2,2-bis[4-(3-methacryloyloxy-2-hydroxypropoxy)phenyl]propane

D-2.6E: 2,2-bis(4-methacryloyloxypolyethoxyphenyl)propane (average addition molar number of ethoxy groups: 2.6)

TEGDMA: triethylene glycol dimethacrylate

THF-MA: tetrahydrofurfuryl methacrylate

[Organic Peroxide (D)]

THP: 1,1,3,3-tetramethylbutyl hydroperoxide

BPB: tert-butyl peroxybenzoate

[Reducing Agent (E)]

PyTU: 1-(2-pyridyl)-2-thiourea

DMETU: 4,4-dimethylethylenethiourea

[Phosphine Compound (F)]

BPFPP: bis(pentafluorophenyl)phenylphosphine

TPFPP: tris(pentafluorophenyl)phosphine

TFPP: tris(4-fluorophenyl)phosphine

[Photopolymerization Initiator (G)]

CQ: dl-camphorquinone
BAPO: bis(2,4,6-trimethylbenzoyl)phenylphosphine oxide

[Polymerization Accelerator]

PDE: ethyl 4-(N,N-dimethylamino)benzoate

[Filler (H)]

Surface-treated barium glass:

Barium glass (product code: "E-3000", available from Esstech, Inc.) was pulverized with a ball mill to provide barium glass powder. The resulting barium glass powder was measured for the average particle diameter with a laser diffraction particle diameter distribution analyzer (Model "SALD-2300", available from Shimadzu Corporation), which was 2.4 μm. By an ordinary method, 100 parts by mass of the barium glass powder was surface-treated with 3 parts by mass of γ-methacryloyloxypropyltrimethoxysilane to provide a surface-treated barium glass powder.

Silane-treated quartz powder:

Quartz (available from Maruwa Quartz Co., Ltd.) was pulverized with a ball mill to provide quartz powder having an average particle diameter of approximately 4.5 μm. By an ordinary method, 100 parts by mass of the quartz powder was surface-treated with 3 parts by mass of γ-methacryloyloxypropyltrimethoxysilane to provide a silane-treated quartz powder.

R972: Fine particle silica, available from Nippon Aerosil Co., Ltd., trade name "Aerosil (registered trade name) R972", average particle diameter: 16 nm
Alumina: Aluminum oxide, available from Nippon Aerosil Co., Ltd., trade name "Aeroxide (registered trade name) Alu C", average particle diameter: 20 nm

[Transition Metal Compound]

CuA: Copper(II) acetate

[Polymerization Inhibitor]

BHT: 2,6-di-tert-butyl-4-methylphenol

[pH Modifier]

TTA: Triethanolamine

[Solvent]

Purified water
Ethanol

(Examples 1-1 to 1-14 and 2-1 to 2-5 and Comparative Examples 1-1 to 1-7 and 2-1 to 2-3)

In the components shown in Tables 2 to 4, the components other than the filler were mixed at the blending ratio shown in Tables 2 to 4 at room temperature (25°C) to provide a uniform liquid component, and then the obtained liquid component was then mixed with the filler to provide dental adhesive compositions of Example 1 (Examples 1-1 to 1-14), Example 2 (Examples 2-1 to 2-5), Comparative Example 1 (Comparative Examples 1-1 to 1-7), and Comparative Example

2 (Comparative Examples 2-1 to 2-3).

Subsequently, the dental adhesive compositions were evaluated in the following manner. The results are shown in Tables 2 to 4.

Table 2 shows the evaluation results of the adhesion strength to the dentine for Example 1 and Comparative Example 1. Table 3 shows the evaluation results of the adhesion strength to the enamel for Example 1 and Comparative Example 1. Table 4 shows the evaluation results of the adhesion strength to the dentine for Example 2 and Comparative Example 2.

[Pretreatment of Bovine Tooth used in Adhesion Strength Test]

The labial surface of a bovine mandibular incisor was polished with silicon carbide paper #80 (available from Nihon Kenshi Co., Ltd.) under running water, so as to expose a flat surface of the enamel or the dentin. The exposed flat surface was further polished with silicon carbide paper #1000 (available from Nihon Kenshi Co., Ltd.) under running water. After polishing, the surface was dried by air-blowing water thereon. An adhesive tape having a thickness of approximately 150 μm which has a circular hole having a diameter of 3 mm was adhered to the dried smooth surface, so as to define the adhesion area.

[Adhesion Strength of Dental Adhesive Composition immediately after Preparation to Bovine Tooth (37°C after 1 day)]

The dental adhesive compositions of Examples 1 and 2 and Comparative Examples 1 and 2 each were applied inside the circular hole of the adhesive tape on the bovine tooth specimen having been treated as above, and immediately air-blown, and then light irradiation was performed with a dental light irradiator "Pencure 2000" (available from Morita Corporation) in the normal mode for 10 seconds.

Thereafter, inside the circular hole, Clearfil (registered trade name) AP-X (available from Kuraray Noritake Dental Inc.) as a filling restoration material (composite resin) was further charged for Example 1 and Comparative Example 1, or Panavia (registered trade name) V5 (available from Kuraray Noritake Dental Inc.) as dental resin cement was further charged for Example 2 and Comparative Example 2, which was then covered with a PP film, followed by pressing. Thereafter, light irradiation was further performed for curing with a dental light irradiator ("Pencure 2000", available from Morita Corporation) in the normal mode for 20 seconds for Clearfil (registered trade name) AP-X, or for 10 seconds for Panavia (registered trade name) V5.

The surface of the cured filling restoration material or dental resin cement was sandblasted with alumina powder of 50 μm at a pressure of 0.4 MPa, and on the surface, a stainless steel circular column (diameter: 7 mm, length 2.5 cm) having dental resin cement "SA Luting (registered trade name) Plus Automix" (available from Kuraray Noritake Dental Inc.) mounted thereon was placed to allow the center of the circular hole to agree substantially with the center of the stainless steel circular column, which were adhered by pressing, so as to produce a test specimen A. Five assemblies of the test specimen A were produced.

The test specimen A was allowed to stand at 25°C for 30 minutes, and immersed in distilled water. The test specimen immersed in distilled water was placed in a thermostat chamber retained to 37°C and allowed to stand for 24 hours. The test specimen was measured for the tensile adhesion strength to the bovine tooth enamel and/or dentine after allowing to stand at 37°C for 24 hours. The tensile adhesion strength was measured with a universal testing machine (Autograph AG-1 100 kN, available from Shimadzu Corporation), at a crosshead speed of 2 mm/min. The tensile adhesion strength to the bovine tooth enamel and/or dentine in the tables is the average value of the measured values of the tensile adhesion strength to the bovine tooth dentine after allowing to stand at 37°C for 24 hours of the five test specimens.

[Adhesion Durability of Dental Adhesive Composition to Bovine Tooth (after TC 4000)]

Five test specimens A produced according to the same method as above each were immersed in distilled water at 37°C for 24 hours, and then applied with a thermal cycle load including 4,000 cycles of alternating immersion in a 4°C water bath and a 60°C water bath for 1 minute each, and thus adhesion durability test specimens (after TC 4000) were produced. The test specimens each were measured for the tensile adhesion strength according to the same method as above. The measured value was designated as a value of the adhesion durability (after TC 4000). The value of the tensile adhesion strength in the tables is the average value of the five test specimens.

[Adhesion Strength of Dental Adhesive Composition subjected to Temperature Acceleration (50°C for 8 weeks) to Bovine Tooth (37°C after 1 day)]

In a product container (light shielding container) of "Clearfil (registered trade name) Universal Bond Quick ER" (available from Kuraray Noritake Dental Inc.), 5 mL each of the dental adhesive compositions of Examples 1 and 2 and Comparative Examples 1 and 2 each were charged, which was stored in dark under a dry atmosphere at 50°C for 8 weeks, and then measured for the adhesion strength in the same manner as in the section [Adhesion Strength of Dental Adhesive Composition immediately after Preparation to Bovine Tooth (37°C after 1 day)].

[Table 2]

Table 2

| | | | Example | | | | | | |
|---|---|---|---|---|---|---|---|---|---|
| | | | 1-1 | 1-2 | 1-3 | 1-4 | 1-5 | 1-6 | 1-7 |
| Dental adhesive composition (part by mass) | Acidic group-containing polymerizable monomer (A) | MDP | 15 | 15 | 15 | 15 | 15 | 15 | 15 |
| | | GPDM | | | | | | | |
| | | 4-META | | | | | | | |
| | Polymerizable monomer having no acidic group (C) | Bis-GMA | 42 | 42 | 42 | 42 | 42 | 42 | 42 |
| | | HEMA | 43 | 43 | 43 | 43 | 43 | 43 | 43 |
| | Solvent | Water | 21 | 21 | 21 | 21 | 21 | 21 | 21 |
| | | Ethanol | 21 | 21 | 21 | 21 | 21 | 21 | 21 |
| | Photoredox catalyst (B) | TPT | 0.7 | | | | 0.7 | | |
| | | MDPT | | 0.7 | | | | | |
| | | $Acr^+Mes$ | | | 0.4 | | | 0.4 | 0.4 |
| | | DCA | | | | 0.07 | | | |
| | Photoredox catalyst (B') | EosinY | | | | | | | |
| | | Fluorescein | | | | | | | |
| | Photopolymerization initiator (G) | CQ | 3 | 3 | 3 | 3 | | 3 | 3 |
| | | BAPO | | | | | | | |
| | Polymerization accelerator for photopolymerization initiator | PDE | 3 | 3 | 3 | 3 | 3 | 3 | 3 |
| | pH Modifier | TTA | 1.5 | 1.5 | 1.5 | 1.5 | 1.5 | 1.5 | 1.5 |
| | Organic peroxide (D) | THP | | | | | | | |
| | | BPB | | | | | | | |
| | Reducing agent (E) | DMETU | | | | | | 1.5 | |
| | | PyTU | | | | | | | |
| | Phosphine compound (F) | BPFPP | | | | | | | 1.5 |
| | | TPFPP | | | | | | | |
| | | TFPP | | | | | | | |
| | Polymerization inhibitor | BHT | 0.07 | 0.07 | 0.07 | 0.07 | 0.07 | 0.07 | 0.07 |
| | Filler (H) | R972 | 14 | 14 | 14 | 14 | 14 | 14 | 14 |
| Composite resin coated on coated and cured product of dental adhesive composition | | | AP-X | AP-X | AP-X | AP-X | AP-X | AP-X | AP-X |

(continued)

| | | | | Example | | | | | | |
|---|---|---|---|---|---|---|---|---|---|---|
| | | | | 1-1 | 1-2 | 1-3 | 1-4 | 1-5 | 1-6 | 1-7 |
| Evaluation | Dentine tensile adhesion strength (MPa) | Immediately after preparation | After 1 day at 37°C | 35 | 34 | 29 | 29 | 22 | 34 | 35 |
| | | | After TC 4000 | 34 | 33 | 28 | 27 | 21 | 33 | 35 |
| | | After 8 weeks at 50°C | After 1 day at 37°C | 28 | 33 | 28 | 31 | 21 | 34 | 33 |
| (continued) | | | | | | | | | | |

Table 2 (continued)

| | | | Example | | | | | | |
|---|---|---|---|---|---|---|---|---|---|
| | | | 1-8 | 1-9 | 1-10 | 1-11 | 1-12 | 1-13 | 1-14 |
| Dental adhesive composition (part by mass) | Acidic group-containing polymerizable monomer (A) | MDP | 15 | 15 | 15 | 15 | 15 | | |
| | | GPDM | | | | | | 15 | |
| | | 4-META | | | | | | | 15 |
| | Polymerizable monomer having no acidic group (C) | Bis-GMA | 42 | 42 | 42 | 42 | 42 | 42 | 42 |
| | | HEMA | 43 | 43 | 43 | 43 | 43 | 43 | 43 |
| | Solvent | Water | 21 | 21 | 21 | 21 | 21 | 21 | 21 |
| | | Ethanol | 21 | 21 | 21 | 21 | 21 | 21 | 21 |
| | Photoredox catalyst (B) | TPT | | | | | | | |
| | | MDPT | | | | | | | |
| | | $Acr^+Mes$ | 0.4 | 0.4 | 0.7 | 0.2 | *0.05* | 0.4 | 0.4 |
| | | DCA | | | | | | | |
| | Photoredox catalyst (B') | EosinY | | | | | | | |
| | | Fluorescein | | | | | | | |
| | Photopolymerization initiator (G) | CQ | 3 | 3 | 3 | 3 | 3 | 3 | 3 |
| | | BAPO | | | | | | | |
| | Polymerization accelerator for photopolymerization initiator | PDE | 3 | 3 | 3 | 3 | 3 | 3 | 3 |
| | pH Modifier | TTA | 1.5 | 1.5 | 1.5 | 1.5 | 1.5 | 1.5 | 1.5 |
| | Organic peroxide (D) | THP | | | | | | | |
| | | BPB | | | | | | | |
| | Reducing agent (E) | DMETU | | | | | | | |
| | | PyTU | | | | | | | |
| | Phosphine compound (F) | BPFPP | | | | | | | |
| | | TPFPP | 1.5 | | | | | | |
| | | TFPP | | 1.5 | | | | | |
| | Polymerization inhibitor | BHT | 0.07 | 0.07 | 0.07 | 0.07 | 0.07 | 0.07 | 0.07 |
| | Filler (H) | R972 | 14 | 14 | 14 | 14 | 14 | 14 | 14 |

(continued)

| | | | | Example | | | | | | |
|---|---|---|---|---|---|---|---|---|---|---|
| | | | | 1-8 | 1-9 | 1-10 | 1-11 | 1-12 | 1-13 | 1-14 |
| Composite resin coated on coated and cured product of dental adhesive composition | | | | AP-X | AP-X | AP-X | AP-X | AP-X | AP-X | AP-X |
| Evaluation | Dentine tensile adhesion strength (MPa) | Immediately after preparation | After 1 day at 37°C | 33 | 32 | 25 | 29 | 23 | 28 | 26 |
| | | | After TC 4000 | 34 | 33 | 24 | 27 | 23 | 27 | 26 |
| | | After 8 weeks at 50°C | After 1 day at 37°C | 33 | 32 | 24 | 28 | 22 | 27 | 24 |
| (continued) | | | | | | | | | | |

Table 2 (continued)

| | | | | Comparative Example | | | | | | |
|---|---|---|---|---|---|---|---|---|---|---|
| | | | | 1-1 | 1-2 | 1-3 | 1-4 | 1-5 | 1-6 | 1-7 |
| Dental adhesive composition (part by mass) | Acidic group-containing polymerizable monomer (A) | | MDP | 15 | 15 | 15 | 15 | 15 | 15 | 0 |
| | | | GPDM | | | | | | | |
| | | | 4-META | | | | | | | |
| | Polymerizable monomer having no acidic group (C) | | Bis-GMA | 42 | 42 | 42 | 42 | 42 | 42 | 42 |
| | | | HEMA | 43 | 43 | 43 | 43 | 43 | 43 | 58 |
| | Solvent | | Water | 21 | 21 | 21 | 21 | 21 | 21 | 21 |
| | | | Ethanol | 21 | 21 | 21 | 21 | 21 | 21 | 21 |
| | Photoredox catalyst (B) | | TPT | | | | | | | 0.7 |
| | | | MDPT | | | | | | | |
| | | | $Acr^+Mes$ | | | | | | | |
| | | | DCA | | | | | | | |
| | Photoredox catalyst (B') | | EosinY | | | | | 0.7 | | |
| | | | Fluorescein | | | | | | 0.7 | |
| | Photopolymerization initiator (G) | | CQ | 3 | 3 | 3 | 3 | 3 | 3 | 3 |
| | | | BAPO | | | *0.5* | *0.5* | | | |
| | Polymerization accelerator for photopolymerization initiator | | PDE | 3 | 3 | 3 | 3 | 3 | 3 | 3 |
| | pH Modifier | | TTA | 1.5 | 1.5 | 1.5 | 1.5 | 1.5 | 1.5 | 1.5 |
| | Organic peroxide (D) | | THP | | 3 | | | | | |
| | | | BPB | | | | | | | |
| | Reducing agent (E) | | DMETU | | | 1.5 | | | | |
| | | | PyTU | | | | | | | |
| | Phosphine compound (F) | | BPFPP | | | | 1.5 | | | |
| | | | TPFPP | | | | | | | |
| | | | TFPP | | | | | | | |
| | Polymerization inhibitor | | BHT | 0.07 | 0.07 | 0.07 | 0.07 | 0.07 | 0.07 | 0.07 |
| | Filler (H) | | R972 | 14 | 14 | 14 | 14 | 14 | 14 | 14 |
| Composite resin coated on coated and cured product of dental adhesive composition | | | | AP-X | AP-X | AP-X | AP-X | AP-X | AP-X | AP-X |
| Evaluation | Dentine tensile adhesion strength (MPa) | Immediately after preparation | After 1 day at 37°C | 18 | 23 | 18 | 19 | 17 | 15 | 4 |
| | | | After TC 4000 | 17 | 20 | 17 | 17 | 16 | 14 | 3 |
| | | After 8 weeks at 50°C | After 1 day at 37°C | 17 | solidified | 14 | 16 | 16 | 14 | 3 |

Table 3

| | | | | Example | | | | | |
|---|---|---|---|---|---|---|---|---|---|
| | | | | 1-1 | 1-2 | 1-3 | 1-4 | 1-6 | 1-7 |
| Dental adhesive composition (part by mass) | Acidic group-containing polymerizable monomer (A) | | MDP | 15 | 15 | 15 | 15 | 15 | 15 |
| | | | GPDM | | | | | | |
| | | | 4-META | | | | | | |
| | Polymerizable monomer having no acidic group (C) | | Bis-GMA | 42 | 42 | 42 | 42 | 42 | 42 |
| | | | HEMA | 43 | 43 | 43 | 43 | 43 | 43 |
| | Solvent | | Water | 21 | 21 | 21 | 21 | 21 | 21 |
| | | | Ethanol | 21 | 21 | 21 | 21 | 21 | 21 |
| | Photoredox catalyst (B) | | TPT | 0.7 | | | | | |
| | | | MDPT | | 0.7 | | | | |
| | | | $Acr^+Mes$ | | | 0.4 | | 0.4 | 0.4 |
| | | | DCA | | | | 0.07 | | |
| | Photoredox catalyst (B') | | EosinY | | | | | | |
| | | | Fluorescein | | | | | | |
| | Photopolymerization initiator (G) | | CQ | 3 | 3 | 3 | 3 | 3 | 3 |
| | | | BAPO | | | | | | |
| | Polymerization accelerator for photopolymerization initiator | | PDE | 3 | 3 | 3 | 3 | 3 | 3 |
| | pH Modifier | | TTA | 1.5 | 1.5 | 1.5 | 1.5 | 1.5 | 1.5 |
| | Organic peroxide (D) | | THP | | | | | | |
| | | | BPB | | | | | | |
| | Reducing agent (E) | | DMETU | | | | | 1.5 | |
| | | | PyTU | | | | | | |
| | Phosphine compound (F) | | BPFPP | | | | | | 1.5 |
| | | | TPFPP | | | | | | |
| | | | TFPP | | | | | | |
| | Polymerization inhibitor | | BHT | 0.07 | 0.07 | 0.07 | 0.07 | 0.07 | 0.07 |
| | Filler (H) | | R972 | 14 | 14 | 14 | 14 | 14 | 14 |
| Composite resin coated on coated and cured product of dental adhesive composition | | | | AP-X | AP-X | AP-X | AP-X | AP-X | AP-X |
| Evaluation | Enamel tensile adhesion strength (MPa) | Immediately after preparation | After 1 day at 37°C | 24 | 22 | 22 | 21 | 24 | 22 |
| | | | After TC 4000 | 20 | 20 | 21 | 20 | 22 | 20 |
| | | After 8 weeks at 50°C | After 1 day at 37°C | 18 | 22 | 19 | 21 | 23 | 18 |
| (continued) | | | | | | | | | |

Table 3 (continued)

| | | | Comparative Example | | | | | | |
|---|---|---|---|---|---|---|---|---|---|
| | | | 1-1 | 1-2 | 1-3 | 1-4 | 1-5 | 1-6 | 1-7 |
| Dental adhesive composition (part by mass) | Acidic group-containing polymerizable monomer (A) | MDP | 15 | 15 | 15 | 15 | 15 | 15 | 0 |
| | | GPDM | | | | | | | |
| | | 4-META | | | | | | | |
| | Polymerizable monomer having no acidic group (C) | Bis-GMA | 42 | 42 | 42 | 42 | 42 | 42 | 42 |
| | | HEMA | 43 | 43 | 43 | 43 | 43 | 43 | 58 |
| | Solvent | Water | 21 | 21 | 21 | 21 | 21 | 21 | 21 |
| | | Ethanol | 21 | 21 | 21 | 21 | 21 | 21 | 21 |
| | Photoredox catalyst (B) | TPT | | | | | | | 0.7 |
| | | MDPT | | | | | | | |
| | | $Acr^+Mes$ | | | | | | | |
| | | DCA | | | | | | | |
| | Photoredox catalyst (B') | EosinY | | | | | 0.7 | | |
| | | Fluorescein | | | | | | 0.7 | |
| | Photopolymerization initiator (G) | CQ | 3 | 3 | 3 | 3 | 3 | 3 | 3 |
| | | BAPO | | | *0.5* | *0.5* | | | |
| | Polymerization accelerator for photopolymerization initiator | PDE | 3 | 3 | 3 | 3 | 3 | 3 | 3 |
| | pH Modifier | TTA | 1.5 | 1.5 | 1.5 | 1.5 | 1.5 | 1.5 | 1.5 |
| | Organic peroxide (D) | THP | | 3 | | | | | |
| | | BPB | | | | | | | |
| | Reducing agent (E) | DMETU | | | 1.5 | | | | |
| | | PyTU | | | | | | | |
| | Phosphine compound (F) | BPFPP | | | | 1.5 | | | |
| | | TPFPP | | | | | | | |
| | | TFPP | | | | | | | |
| | Polymerization inhibitor | BHT | 0.07 | 0.07 | 0.07 | 0.07 | 0.07 | 0.07 | 0.07 |
| | Filler (H) | R972 | 14 | 14 | 14 | 14 | 14 | 14 | 14 |
| Composite resin coated on coated and cured product of dental adhesive composition | | | AP-X | AP-X | AP-X | AP-X | AP-X | AP-X | AP-X |
| Evaluation | Enamel tensile adhesion strength (MPa) | Immediately after preparation / After 1 day at 37°C | 17 | 20 | 19 | 20 | 14 | 12 | 2 |
| | | Immediately after preparation / After TC 4000 | 16 | 19 | 18 | 18 | 13 | 12 | 1 |
| | | After 8 weeks at 50°C / After 1 day at 37°C | 15 | solidified | 15 | 20 | 13 | 13 | 2 |

Table 4

| | | | Example | | | | |
|---|---|---|---|---|---|---|---|
| | | | 2-1 | 2-2 | 2-3 | 2-4 | 2-5 |
| Dental adhesive composition (part by mass) | Acidic group-containing polymerizable monomer (A) | MDP | 15 | 15 | 15 | 15 | 15 |
| | Polymerizable monomer having no acidic group (C) | Bis-GMA | 42 | 42 | 42 | 42 | 42 |
| | | HEMA | 43 | 43 | 43 | 43 | 43 |
| | Solvent | Water | 21 | 21 | 21 | 21 | 21 |
| | | Ethanol | 21 | 21 | 21 | 21 | 21 |
| | Photoredox catalyst (B) | TPT | 0.7 | | | 0.7 | |
| | | $Acr^+Mes$ | | 0.4 | | | 0.4 |
| | | DCA | | | 0.07 | | |
| | Photopolymerization initiator (G) | CQ | 3 | 3 | 3 | | 3 |
| | | BAPO | | | | | 0.7 |
| | Polymerization accelerator for photopolymerization initiator | PDE | 3 | 3 | 3 | 3 | 3 |
| | pH Modifier | TTA | 1.5 | 1.5 | 1.5 | 1.5 | 1.5 |
| | Organic peroxide (D) | THP | | | | | |
| | Reducing agent (E) | DMETU | | | | | 1.5 |
| | Polymerization inhibitor | BHT | 0.07 | 0.07 | 0.07 | 0.07 | 0.07 |
| | Filler (H) | R972 | 14 | 14 | 14 | 14 | 14 |
| Cement coated on coated and cured product of dental adhesive composition | | | Panavia V5 | Panavia V5 | Panavia V5 | Panavia V5 | Panavia V5 |
| Evaluation | Dentine tensile adhesion strength (MPa) | Immediately after preparation | 35 | 34 | 30 | 27 | 34 |
| | | After 8 weeks at 50°C | 29 | 32 | 31 | 24 | 33 |
| (continued) | | | | | | | |

Table 4 (continued)

| | | | Com parative Example | | |
|---|---|---|---|---|---|
| | | | 2-1 | 2-2 | 2-3 |
| Dental adhesive composition (part by mass) | Acidic group-containing polymerizable monomer (A) | MDP | 15 | 15 | 15 |
| | Polymerizable monomer having no acidic group (C) | Bis-GMA | 42 | 42 | 42 |
| | | HEMA | 43 | 43 | 43 |
| | Solvent | Water | 21 | 21 | 21 |
| | | Ethanol | 21 | 21 | 21 |
| | Photoredox catalyst (B) | TPT | | | |
| | | $Acr^+Mes$ | | | |
| | | DCA | | | |
| | Photopolymerization initiator (G) | CQ | 3 | 3 | 3 |
| | | BAPO | | | 0.7 |
| | Polymerization accelerator for photopolymerization initiator | PDE | 3 | 3 | 3 |
| | pH Modifier | TTA | 1.5 | 1.5 | 1.5 |
| | Organic peroxide (D) | THP | | 3 | |
| | Reducing agent (E) | DMETU | | | 1.5 |
| | Polymerization inhibitor | BHT | 0.07 | 0.07 | 0.07 |
| | Filler (H) | R972 | 14 | 14 | 14 |
| Cement coated on coated and cured product of dental adhesive composition | | | Panavia V5 | Panavia V5 | Panavia V5 |
| Evaluation | Dentine tensile adhesion strength (MPa) | Immediately after preparation | 21 | 24 | 22 |
| | | After 8 weeks at 50°C | 22 | solidified | 21 |

As shown in Tables 2 to 4, the dental adhesive compositions of Examples resulted in that the composition used immediately after preparation was excellent in adhesion strength to the bovine tooth. The dental adhesive compositions after storing (at 50°C for 8 weeks) showed a small change in adhesion strength to the bovine tooth as compared to immediately after preparation. The evaluation of the adhesion strength after TC 4000 in Tables 2 and 3 showed the excellent adhesion durability.

Comparative Examples 1-1 to 1-6 and Comparative Examples 2-1 to 2-3 are dental adhesive compositions that do not contain the photoredox catalyst (B).

Comparative Example 1-1 and Comparative Example 2-1 showed lower adhesion strength than Examples.

Comparative Examples 1-2, 1-3, 1-4, 2-2, and 2-3 are dental adhesive compositions that do not contain the photoredox catalyst (B) and contains the organic peroxide (D) (Comparative Examples 1-2 and 2-2), the reducing agent (E) (Comparative Examples 1-3 and 2-3), or the phosphine compound (F) (Comparative Example 1-4) added thereto. The dental adhesive compositions of Comparative Examples 1-2 and 2-2 had slight effects observed, but the composition had low storage stability, and the composition was solidified after storing (at 50°C for 8 weeks). Therefore, adhesion strength was unable to evaluate. The dental adhesive compositions of Comparative Examples 1-3, 1-4, and 2-3 did not contain the photoredox catalyst (B), and therefore were insufficient in the adhesiveness enhancing effect even though the additives were added thereto.

The dental adhesive compositions of Comparative Examples 1-5 and 1-6 are compositions having added thereto the photoredox catalyst (B') that does not satisfy the requirement of a singlet excited state reduction potential $E^{S1}_{red}$ of 1.5 V vs SCE or more. The dental adhesive compositions showed no adhesion enhancing effect.

Comparative Example 1-7 did not contain the polymerizable monomer (A) having an acidic group, and was inferior in adhesion strength.

(Examples 3-1, 3-2, 4-1, and 4-2 and Comparative Examples 3-1 and 4-1)

In the components of the first parts and the second parts in Table 5 and the components described in Table 6, the components other than the filler were mixed at the blending ratio shown in Tables 5 and 6 at room temperature (25°C) to provide a uniform liquid component, and then the obtained liquid component was mixed with the filler to provide the first parts and the second parts in Table 5 and the dental adhesive compositions described in Table 6, respectively. The dental adhesive compositions (dental self-adhesive resin cement) of Example 3 (Examples 3-1 and 3-2) and Comparative Example 3 (Comparative Example 3-1) each were formed of the first part and the second part in Table 5, which were mixed in equal volumes immediately before use and subjected to the test. The dental adhesive compositions (dental adhesive composition composite resins) of Example 4 (Examples 4-1 and 4-2) and Comparative Example 4 (Comparative Example 4-1) each was a one-component type, and therefore was used directly.

Subsequently, the dental adhesive compositions were evaluated in the following manner. The results are shown in Tables 5 and 6.

[Pretreatment of Bovine Tooth used in Adhesion Strength Test]

The bovine tooth was pre-treated in the manner described above.

[Adhesion Strength of Dental Resin Cement and Dental Self-adhesive Composite Resin immediately after Preparation to Bovine Tooth (37°C after 1 day)]

The dental adhesive compositions of Examples 3 and 4 and Comparative Examples 3 and 4 each were applied inside the circular hole of the adhesive tape, and covered with a PP film and pressed therewith for fixing the thickness, and then light irradiation was performed with "Pencure 2000" (available from Morita Corporation) in the normal mode for 10 seconds.

Thereafter, the surface of the cured dental adhesive composition was sandblasted with alumina powder of 50 μm at a pressure of 0.4 MPa, and on the surface, a stainless steel circular column (diameter: 7 mm, length 2.5 cm) having dental resin cement "SA Luting (registered trade name) Plus Automix" (available from Kuraray Noritake Dental Inc.) mounted thereon was placed to allow the center of the circular hole to agree substantially with the center of the stainless steel circular column, which were adhered by pressing, so as to produce a test specimen B. Five assemblies of the test specimen B were produced.

The test specimen B was allowed to stand at 25°C for 30 minutes, and immersed in distilled water. The test specimen immersed in distilled water was placed in a thermostat chamber retained to 37°C and allowed to stand for 24 hours. The test specimen was measured for the tensile adhesion strength to the bovine tooth dentine after allowing to stand at 37°C for 24 hours. The tensile adhesion strength to the bovine tooth dentine in the tables is the average value of the measured values of the tensile adhesion strength to the bovine tooth dentine after allowing to stand at 37°C for 24 hours of the five test specimens.

[Adhesion Strength of Dental Adhesive Composition subjected to Temperature Acceleration (60°C for 3 weeks) to Bovine Tooth (37°C after 1 day)]

In a product container (light shielding container) of "Clearfil (registered trade name) Universal Bond Quick ER" (available from Kuraray Noritake Dental Inc.), 5 mL each of the dental adhesive compositions of Examples 3 and 4 and Comparative Examples 3 and 4 each were charged, which was stored in dark under a dry atmosphere at 60°C for 3 weeks, and then measured for the adhesion strength in the same manner as in the section [Adhesion Strength of Dental Adhesive Composition immediately after Preparation to Bovine Tooth (37°C after 1 day)].

[Table 5]

Table 5

| | | | | Example | | Comparative Example |
|---|---|---|---|---|---|---|
| | | | | 3-1 | 3-2 | 3-1 |
| Dental adhesive composition (part by mass) | First part | Acidic group-containing polymerizable monomer (A) | MDP | 10 | 10 | 10 |
| | | Polymerizable monomer having no acidic group (C) | HEMA | 15 | 15 | 15 |
| | | | Bis-GMA | 20 | 20 | 20 |
| | | | D-2.6E | 30 | 30 | 30 |
| | | | TEGDMA | 25 | 25 | 25 |
| | | Photoredox catalyst (B) | TPT | 0.5 | | |
| | | | $Acr^+Mes$ | | 0.5 | |
| | | Organic peroxide (D) | THP | 3 | 3 | 3 |
| | | pH Modifier | TTA | 2 | 2 | 2 |
| | | Polymerization inhibitor | BHT | 0.15 | 0.15 | 0.15 |
| | | Filler (H) | Surface-treated barium glass | 210 | 210 | 210 |
| | | | R972 | 20 | 20 | 20 |
| | Second part | Polymerizable monomer having no acidic group (C) | D-2.6E | 85 | 85 | 85 |
| | | | TEGDMA | 15 | 15 | 15 |
| | | Reducing agent (E) | PyTU | 0.75 | 0.75 | 0.75 |
| | | Transition metal compound | CuA | 0.008 | 0.008 | 0.008 |
| | | Polymerization inhibitor | BHT | 0.05 | 0.05 | 0.05 |
| | | Filler (H) | Surface-treated barium glass | 225 | 225 | 225 |
| | | | Alumina | 15 | 15 | 15 |
| Evaluation | | Dentine tensile adhesion strength (after 1 day at 37°C) (MPa) | Immediately after preparation | 13.4 | 12.8 | 7.2 |
| | | | After 3 weeks at 60°C | 10.2 | 12.7 | 7.1 |

[Table 6]

Table 6

| | | | Example | | Comparative Example |
|---|---|---|---|---|---|
| | | | 4-1 | 4-2 | 4-1 |
| Dental adhesive composition (part by mass) | Acidic group-containing polymerizable monomer (A) | MDP | 10 | 10 | 10 |
| | Polymerizable monomer having no acidic group (C) | HEMA | 5 | 15 | 15 |
| | | GDMA | 10 | 20 | 20 |
| | | D-2.6E | 50 | 30 | 30 |
| | | THF-MA | 25 | 25 | 25 |
| | Photoredox catalyst (B) | TPT | 0.3 | | |
| | | $Acr^{+}Mes$ | | 0.01 | |
| | Photopolymerization initiator (G) | CQ | 0.2 | 0.2 | 0.2 |
| | | BAPO | 0.3 | 0.3 | 0.3 |
| | Polymerization accelerator | PDE | 0.2 | 0.2 | 0.2 |
| | Polymerization inhibitor | BHT | 0.1 | 0.1 | 0.1 |
| | Filler (H) | Surface-treated quartz powder | 40 | 40 | 40 |
| | | Surface-treated barium glass | 200 | 200 | 200 |
| | | R972 | 10 | 10 | 10 |
| Evaluation | Dentine tensile adhesion strength (after 1 day at 37°C) (MPa) | Immediately after preparation | 12.5 | 11.1 | 7.8 |
| | | After 3 weeks at 60°C | 10.1 | 11.2 | 7.1 |

As shown in Tables 5 and 6, the dental adhesive compositions of Examples resulted in that the composition used immediately after preparation was excellent in adhesion strength to the bovine tooth. The dental adhesive compositions after storing (at 60°C for 3 weeks) showed a small change in adhesion strength to the bovine tooth as compared to immediately after preparation.

Comparative Example 3-1 and Comparative Example 4-1 are dental adhesive compositions that do not contain the photoredox catalyst (B). These dental adhesive compositions showed lower adhesion strength than Examples.

Industrial Applicability

The dental adhesive composition of the present invention can be favorably applied to a dental restorative material and adhesion of a dental prosthesis, such as a crown, an inlay, and a bridge, to the tooth substance in the dental treatment.

## Claims

1. A dental adhesive composition comprising a polymerizable monomer (A) having an acidic group and a photoredox catalyst (B) having a singlet excited state reduction potential $E^{S1}_{red}$ of 1.5 V vs SCE or more.

2. The dental adhesive composition according to claim 1, wherein the photoredox catalyst (B) has a negative singlet excited state oxidation potential $E^{S1}_{ox}$.

3. The dental adhesive composition according to claim 1 or 2, wherein the photoredox catalyst (B) is at least one kind selected from the group consisting of compounds represented by the following formulae (1), (2), and (3):

[Chem. 1]

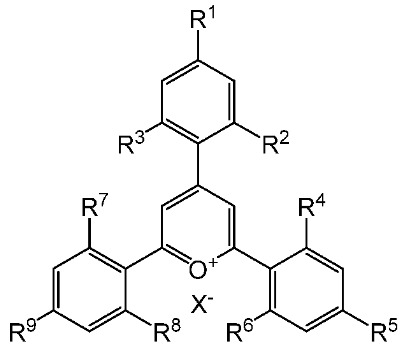

(1)

wherein in the formula (1), $R^1$, $R^2$, $R^3$, $R^4$, $R^5$, $R^6$, $R^7$, $R^8$, and $R^9$ each independently represent a hydrogen atom, an alkyl group having 1 to 10 carbon atoms, an alkoxy group having 1 to 10 carbon atoms, or a halogen atom, and $X^-$ represents $BF_4^-$, $PF_6^-$, $ClO_4^-$, or $HSO_4^-$,

[Chem. 2]

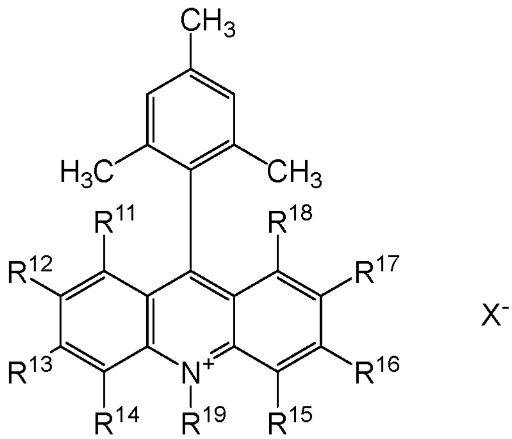

(2)

in the formula (2), $R^{11}$, $R^{13}$, $R^{14}$, $R^{15}$, $R^{16}$, and $R^{18}$ each independently represent a hydrogen atom or an alkyl group having 1 to 10 carbon atoms, $R^{12}$, $R^{17}$, and $R^{19}$ each independently represent a hydrogen atom, an alkyl group having 1 to 10 carbon atoms, or an aryl group having 6 to 30 carbon atoms, and $X^-$ represents $BF_4^-$, $PF_6^-$, $ClO_4^-$, or $HSO_4^-$,

[Chem. 3]

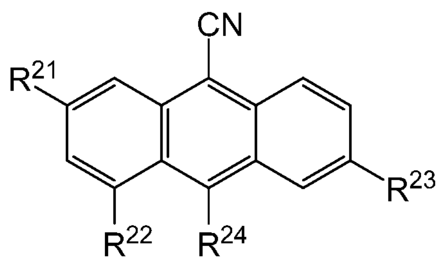

(3)

in the formula (3), $R^{21}$, $R^{22}$, $R^{23}$, and $R^{24}$ each independently represent a hydrogen atom, an alkyl group having 1 to 10 carbon atoms, an alkoxy group having 1 to 10 carbon atoms, a carboxylic acid group having 1 to 10 carbon atoms or an ester thereof, a hydroxy group, a cyano group, or a halogen atom.

**4.** The dental adhesive composition according to claim 3, wherein in the formula (1), $R^1$, $R^2$, and $R^3$ each independently represent an alkyl group having 1 to 4 carbon atoms.

**5.** The dental adhesive composition according to claim 3 or 4, wherein in the formula (1), $R^1$, $R^2$, and $R^3$ each independently represent a methyl group, an isopropyl group, or a tert-butyl group.

**6.** The dental adhesive composition according to any one of claims 1 to 5, wherein the photoredox catalyst (B) has an absorption spectrum including a range of 400 nm or more and 500 nm or less.

**7.** The dental adhesive composition according to any one of claims 1 to 6, wherein the dental adhesive composition further comprises a polymerizable monomer (C) having no acidic group.

**8.** The dental adhesive composition according to any one of claims 1 to 7, wherein the dental adhesive composition further comprises an organic peroxide (D).

**9.** The dental adhesive composition according to any one of claims 1 to 8, wherein the dental adhesive composition further comprises a reducing agent (E).

**10.** The dental adhesive composition according to any one of claims 1 to 9, wherein the dental adhesive composition further comprises a phosphine compound (F) having an electron withdrawing group.

**11.** The dental adhesive composition according to any one of claims 1 to 10, wherein the dental adhesive composition further comprises a photopolymerization initiator (G).

**12.** The dental adhesive composition according to any one of claims 1 to 11, wherein the dental adhesive composition is a one-component type dental bonding material.

**13.** The dental adhesive composition according to any one of claims 1 to 11, wherein the dental adhesive composition is a dental self-adhesive composite resin.

**14.** The dental adhesive composition according to any one of claims 1 to 11, wherein the dental adhesive composition is dental self-adhesive resin cement.

**15.** A kit comprising the dental adhesive composition according to claim 12 and a dental two-paste type composition.

**16.** A kit comprising the dental adhesive composition according to claim 12 and a dental one-paste type composition.

**INTERNATIONAL SEARCH REPORT**

International application No.
**PCT/JP2023/039313**

**A. CLASSIFICATION OF SUBJECT MATTER**

***A61K 6/831***(2020.01)i; ***A61C 13/00***(2006.01)i; ***A61C 13/23***(2006.01)i; ***A61K 6/887***(2020.01)i
FI: A61K6/831; A61K6/887; A61C13/00 C; A61C13/23

According to International Patent Classification (IPC) or to both national classification and IPC

**B. FIELDS SEARCHED**

Minimum documentation searched (classification system followed by classification symbols)

A61K6/831; A61C13/00; A61C13/23; A61K6/887

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | JP 61-60603 A (NOF CORP.) 28 March 1986 (1986-03-28) entire text | 1-16 |
| A | JP 62-45509 A (NOF CORP.) 27 February 1987 (1987-02-27) entire text | 1-16 |
| A | JP 2020-176089 A (KURARAY NORITAKE DENTAL INC.) 29 October 2020 (2020-10-29) entire text | 1-16 |
| A | WO 2010/106903 A1 (KURARAY MEDICAL INC.) 23 September 2010 (2010-09-23) entire text | 1-16 |

☐ Further documents are listed in the continuation of Box C.    ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 December 2023** | **23 January 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/039313**

| Patent document cited in search report | Publication date (day/month/year) | Patent family member(s) | Publication date (day/month/year) |
|---|---|---|---|
| JP 61-60603 A | 28 March 1986 | US 4746685 A | |
| | | US 4824876 A | |
| | | US 4923905 A | |
| | | EP 176777 A2 | |
| JP 62-45509 A | 27 February 1987 | (Family: none) | |
| JP 2020-176089 A | 29 October 2020 | (Family: none) | |
| WO 2010/106903 A1 | 23 September 2010 | US 2012/0016094 A1 | |
| | | EP 2409997 A1 | |
| | | CN 102356097 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008087981 A
- WO 2010106903 A
- WO 2013082337 A
- JP 3057916 B


### Non-patent literature cited in the description


- *Chem. Rev.*, 2016, vol. 116, 10075-10166